(19) Europäisches Patentamt
European Patent Office
Office européen des brevets

(11) **EP 4 158 642 B1**

(12) **EUROPEAN PATENT SPECIFICATION**

(45) Date of publication and mention
of the grant of the patent:
**24.12.2025 Bulletin 2025/52**

(51) International Patent Classification (IPC):
**G16H 20/17** (2018.01)    **G16H 40/63** (2018.01)
**A61M 5/00** (2006.01)    G16H 20/40 (2018.01)
**A61M 5/145** (2006.01)

(21) Application number: **21735518.9**

(22) Date of filing: **01.06.2021**

(52) Cooperative Patent Classification (CPC):
**G16H 20/17; A61M 5/14546; G16H 40/63;**
A61M 5/007; A61M 5/1452; A61M 2205/332;
G16H 20/40

(86) International application number:
**PCT/US2021/035273**

(87) International publication number:
**WO 2021/247595 (09.12.2021 Gazette 2021/49)**

(54) **METHOD FOR CONTROLLING A FLUID INJECTION SYSTEM BASED ON HYDRAULIC RESISTANCE**

VERFAHREN ZUR STEUERUNG EINES FLÜSSIGKEITSEINSPRITZSYSTEMS AUF BASIS VON HYDRAULISCHEM WIDERSTAND

PROCÉDÉ POUR COMMANDER UN SYSTÈME D'INJECTION DE FLUIDE SUR LA BASE D'UNE RÉSISTANCE HYDRAULIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.06.2020 US 202062704893 P**

(43) Date of publication of application:
**05.04.2023 Bulletin 2023/14**

(73) Proprietor: **Bayer HealthCare LLC**
**Whippany, NJ 07981 (US)**

(72) Inventors:
• **LEE, Randy**
**Pittsburgh, Pennsylvania 15218 (US)**
• **DELBRUGGE, Gerald**
**Clearfield, Pennsylvania 16830 (US)**
• **SPOHN, Michael**
**Fenelton, Pennsylvania 16034 (US)**
• **SCUTT, Christopher**
**Murrysville, Pennsylvania 15668 (US)**
• **MCGUIRE, Matthew**
**Pittsburgh, Pennsylvania 15227 (US)**
• **SHINDE, Abhishek**
**Monroeville, Pennsylvania 15146 (US)**
• **NAPLES, Andrew**
**Mars, Pennsylvania 16046 (US)**
• **MCDERMOTT, Michael**
**10179 Berlin (DE)**
• **UBER III, Arthur**
**Pittsburgh, Pennsylvania 15208 (US)**

(74) Representative: **BIP Patents**
**c/o Bayer Intellectual Property GmbH**
**Alfred-Nobel-Straße 50**
**40789 Monheim am Rhein (DE)**

(56) References cited:
EP-A2- 1 898 210    WO-A1-2014/160326
WO-A1-2018/089882    US-A1- 2003 041 652
US-A1- 2019 307 953    US-A1- 2020 121 860

**Description**

CROSS-REFERENCE TO RELATED APPLICATIONS

This application claims the benefit of U.S. Provisional Application No. 62/704,893, filed June 2, 2020.

BACKGROUND

1. <u>Field</u>

This disclosure relates generally to systems, devices, products, apparatus, and methods for controlling a fluid injection system, and in various embodiments, to a system, product, and method for controlling a fluid injection system based on motor controller gains of a motor of a powered fluid injector in the fluid injection system.

2. <u>Technical Considerations</u>

A fluid injection device (e.g., a medical fluid delivery device), such as a powered injector device may be used by a medical practitioner, such as a physician, in a medical diagnostic procedure and/or a medical therapeutic procedure. For example, the medical practitioner may use the fluid injection device to inject a patient with one or more medical fluids. The fluid injection device may be used for pressurized injection of a medical fluid, such as a radiological contrast fluid (e.g., a contrast agent, a radiocontrast agent, etc.), and/or a flushing agent, such as saline, in a medical imaging procedure such as cardiovascular angiography (CV), computed tomography (CT), ultrasound, magnetic resonance imaging (MRI), and positron emission tomography (PET). In some instances, the fluid injection device is designed to deliver a preset amount of a medical fluid at a preset flow rate.

US 2020/121860 A1 discloses systems, devices, products, apparatus, and methods for controlling a fluid injection system based on motor controller gains of a motor of a powered fluid injector in the fluid injection system.

SUMMARY

Accordingly, provided are systems, devices, products, apparatus, and/or methods for controlling a fluid injection system that improves operation of a motor of a powered fluid injector in the fluid injection system.

These and other features and characteristics of the present disclosure, as well as the methods of operation and functions of the related elements of structures and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the present disclosure. As used in the specification and the claims, the singular form of "a," "an," and "the" include plural referents unless the context clearly dictates otherwise.

BRIEF DESCRIPTION OF THE DRAWINGS

Additional advantages and details of the present disclosure are explained in greater detail below with reference to the exemplary embodiments that are illustrated in the accompanying schematic figures, in which:

**FIG. 1** is a diagram of a non-limiting embodiment of an environment in which systems, devices, products, apparatus, and/or methods, described herein, may be implemented according to the principles of the present disclosure;
**FIG. 2** is a diagram of a non-limiting embodiment of components of one or more systems or one or more devices of **FIG. 1**;
**FIG. 3** is a flowchart of a non-limiting embodiment of a process for controlling a fluid injection system;
**FIG. 4** a diagram of a non-limiting embodiment of a powered fluid injector;
**FIG. 5** is a schematic diagram of a powered fluid injector along with components for delivering medical fluid to a patient;
**FIG. 6** is a diagram of a process for optimizing fluid delivery in a fluid injection system;
**FIG. 7** is a diagram of components of the powered fluid injector of **FIG. 5;**
**FIG. 8A** is a graph of flow rate of a medical fluid versus time for a constant pressure and **FIG. 8B** is graph of pressure versus time for a constant flow rate of a medical fluid; and
**FIG. 9** is a diagram of a plurality of plots of lines representing plunger force versus fill flow rate for values of viscosity of contrast.

DETAILED DESCRIPTION

For purposes of the description hereinafter, the terms "end," "upper," "lower," "right," "left," "vertical," "horizontal," "top," "bottom," "lateral," "longitudinal," and derivatives thereof shall relate to the present disclosure as it is oriented in the drawing figures. However, it is to be understood that the present disclosure may assume various alternative variations and step sequences, except where expressly specified to the contrary. It is also to be understood that the specific devices and processes illustrated in the attached drawings, and described in the following specification, are simply exemplary embodiments of the present disclosure. Hence, specific dimensions and other physical characteristics related to the embodiments of the embodiments disclosed herein are not to be considered as limiting unless otherwise indicated.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise. In appropriate context, the phrase "based on" may be synonymous with the phrase "in response to," which may indicate a condition (e.g., a triggering condition) upon the occurrence of which a step, operation, function, and/or the like, is performed.

In some non-limiting embodiments, numbers used in the specification and claims may be understood as being modified in all instances by the term "about". The terms "approximately", "about", and "substantially" may mean a range of plus or minus ten percent of the stated value.

As used herein, the term "at least one of" is synonymous with "one or more of". For example, the phrase "at least one of A, B, and C" means any one of A, B, and C, or any combination of any two or more of A, B, and C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of". For example, the phrase "at least two of D, E, and F" means any combination of any two or more of D, E, and F.

When used in relation to a fluid reservoir, such as a syringe, the term "distal" may refer to a portion of the fluid reservoir nearest to a patient. When used in relation to a fluid reservoir, such as a syringe, the term "proximal" may refer to a portion of the fluid reservoir nearest to the injector system.

As used herein, the terms "communication" and "communicate" may refer to the reception, receipt, transmission, transfer, provision, and/or the like of information (e.g., data, signals, messages, instructions, commands, and/or the like). This may refer to a direct or indirect connection that is wired and/or wireless in nature. Additionally, two units may be in communication with each other even though the information transmitted may be modified, processed, relayed, and/or routed between the first and second unit. For example, a first unit may be in communication with a second unit even though the first unit passively receives information and does not actively transmit information to the second unit. As another example, a first unit may be in communication with a second unit if at least one intermediary unit (e.g., a third unit located between the first unit and the second unit) processes information received from the first unit and communicates the processed information to the second unit. In some non-limiting embodiments, a message may refer to a network packet (e.g., a data packet and/or the like) that includes data.

As used herein, the term "system" may refer to one or more computing devices or combinations of computing devices such as, but not limited to, processors, servers, client devices, software applications, and/or other like components as well as one or more mechanical features or devices operated, at least in part, by the computing device(s). In addition, reference to "a server" or "a processor," as used herein, may refer to a previously-recited server and/or processor that is recited as performing a previous step or function, a different server and/or processor, and/or a combination of servers and/or processors. For example, as used in the specification and the claims, a first server and/or a first processor that is recited as performing a first step or function may refer to the same or different server and/or a processor recited as performing a second step or function.

Some non-limiting embodiments are described herein in connection with thresholds. As used herein, satisfying a threshold may refer to a value being greater than the threshold or greater than or equal to the threshold, less than the threshold or less than or equal to the threshold.

As used herein, the term contrast includes solutions of a contrast media and mixtures of the contrast media with a dilutant, such as saline, during a dual flow injection procedure where a selected ratio of contrast and saline is delivered to a patient, for example by concomitant operation of a contrast pump and a saline pump as selected fluid delivery rates with suitable mixing. During a medical imaging procedure, a fluid injection procedure may be conducted where contrast media is injected into the body of a patient using a powered fluid injector. The contrast media may increase the contrast of structures, organs, and/or fluids within the body so that images can be produced of the body. In some instances, the contrast media may be injected through a catheter (e.g., an intravenous catheter or an intraarterial catheter) that is inserted into the body of the patient. During a fluid injection procedure, when contrast media is injected into the body of the patient, the fluid injection system may encounter hydraulic resistance, which may be resistance of the contrast media, flushing fluid, or other medical fluid to flow in a fluid path from a reservoir of the powered fluid injector through the catheter and into the vasculature system of a patient. The hydraulic resistance produced by a catheter depends primarily on factors such as the catheter's length and inner diameter (ID), along with the viscosity, density, and/or flow rate of the medical fluid moving through the catheter. In some instances, hydraulic resistance may increase by increasing one or more of the length of the catheter and/or the density, flow rate, and/or viscosity of the medical fluid moving through the catheter and hydraulic resistance may decrease by one or more of increasing the ID of the catheter, decreasing the length of the catheter and/or the decreasing density, flow rate, and/or viscosity of the medical fluid.

Hydraulic resistance experienced by the fluid injection system may affect control because systems may use proportional, integral, and derivative (PID) control. Motor control gains associated with the PID control may include values that will confer reasonable motor performance over a wide variety of equipment and programmed protocol combinations but those values may work well for extreme values of hydraulic resistance experienced during some injection procedures. In certain embodiments, aspects that may be challenging for accurate control of the motor include, but are not limited to, small bore catheters and/or highly viscous fluids; such conditions may require relatively large pressures to move a medical fluid through the catheter at a given flow rate. A result of motor control inaccuracy may be that the motor overshoots or undershoots on the target pressure, which may either raise safety issues or sacrifice achievable pressure and flow rate of a medical fluid.

Non-limiting embodiments of the present disclosure are directed to systems, devices, products, apparatus, and/or methods for controlling a fluid injection system. In some non-limiting embodiments, an injection management system may include at least one processor programmed or configured to determine at least one characteristic of a power injection protocol, wherein the at least one characteristic of the power injection protocol is associated with a medical fluid involved in the power injection protocol, may determine an estimated value of viscosity of the medical fluid based on the at least one characteristic of the power injection protocol, may calculate a hydraulic resistance score based on the estimated value of viscosity of the medical fluid, and may determine one or more motor controller gains of a motor of a powered fluid injector in the power injection protocol based on the hydraulic resistance score, wherein the motor is configured to reciprocally drive a piston of a fluid injector, for example to reciprocally move a plunger of a syringe to take in a medical fluid or deliver the medical fluid to a patient through a fluid path, such as a fluid line and catheter. When determining the at least one characteristic of the power injection protocol, the at least one processor is programmed or configured to determine a steady state characteristic of saline flowing through an orifice of a fluid path of the powered fluid injector, such as a lumen of a catheter, a lumen of a tube, a distal nozzle of a syringe or a tubing connector of a peristaltic pump, determine a steady state characteristic of a contrast flowing through the orifice; and calculate a characteristic ratio, wherein the characteristic ratio is a ratio of the steady state characteristic of saline to the steady state characteristic of the contrast. Further, when determining the estimated value of viscosity of the medical fluid, the at least one processor is programmed or configured to determine an estimated value of viscosity of the medical fluid based on the characteristic ratio, and when calculating the hydraulic resistance score, the at least one processor is programmed or configured to calculate the hydraulic resistance score based on the estimated value of viscosity of the medical fluid. While many of the non-limiting embodiments are represented by using a contrast media as the medical fluid, it is to be understood that other medical fluids may also be used as the injected fluid medium and the various embodiments of the disclosure herein is not limited to imaging contrast agents.

In some non-limiting embodiments, the at least one processor may be further programmed or configured to implement the one or more motor controller gains on the motor of the powered fluid injector. In some non-limiting embodiments, the motor is a motor of a pump of the powered fluid injector, and the pump of the powered fluid injector may comprise a piston configured for driving a plunger of a syringe; or may rotate a rotatable component of a peristaltic pump. In some non-limiting embodiments, the at least one processor may be further programmed or configured to determine whether the characteristic ratio satisfies a threshold value, and when calculating the hydraulic resistance score, the at least one processor may be programmed or configured to calculate the hydraulic resistance score based on determining that the characteristic ratio satisfies the threshold value. In some non-limiting embodiments, when determining whether the characteristic ratio satisfies the threshold value, the at least one processor is programmed or configured to determine whether the characteristic ratio satisfies a threshold value equal to 1.

In some non-limiting embodiments, the steady state characteristic of saline flowing through the orifice, such as a lumen of a catheter, a lumen of a tube, a distal nozzle of a syringe or a tubing connector of a peristaltic pump, comprises a steady state flow rate of saline flowing through the orifice based on a constant pressure of saline and the steady state characteristic of the contrast flowing through the orifice comprises a steady state flow rate of the contrast flowing through the orifice based on a constant pressure of the contrast, and the characteristic ratio comprises a flow rate ratio, wherein the flow rate ratio is a ratio of the steady state flow rate of saline to the steady state flow rate of the contrast. When calculating the characteristic ratio, the at least one processor is programmed or configured to calculate the flow rate ratio based on the steady state flow rate of saline and the steady state flow rate of the contrast. In some non-limiting embodiments, the steady state characteristic of saline flowing through the orifice comprises a steady state pressure of saline flowing through the orifice based on a constant flow rate of saline, and the steady state characteristic of the contrast flowing through the orifice comprises a steady state pressure of the contrast flowing through the orifice based on a constant flow rate of the contrast. The characteristic ratio comprises a pressure ratio, wherein the pressure ratio is a ratio of the steady state pressure of saline to the steady state pressure of the contrast, and when calculating the characteristic ratio, the at least one processor is programmed or configured to calculate the pressure ratio based on the steady state pressure of saline and the steady state pressure of the contrast. In some non-limiting embodiments, the at least one processor may be further programmed or configured to determine a value of hydraulic capacitance of the medical fluid involved in the power injection protocol, and when determining the motor controller gains, the at least one processor may be programmed or configured to determine the motor controller gains based on the value of hydraulic capacitance of the medical fluid involved in the power injection protocol.

In some non-limiting embodiments, when determining the at least one characteristic of the power injection protocol, the at least one processor may be programmed or configured to determine a first pressure of the power injection protocol when the power injection protocol is in a vacuum fill state, i.e., closed off from receiving the medical fluid in a fluid reservoir, determine a second pressure of the power injection protocol when the power injection protocol is in a normal fill state, i.e., open to receiving medical fluid in the fluid reservoir, and calculate a difference in pressure of the power injection protocol between the vacuum fill state and the normal fill state based on the first pressure of the power injection protocol and the second pressure of the power injection protocol. When determining the estimated value of viscosity of the medical fluid, the at least one processor may be programmed or configured to determine the estimated value of viscosity of the medical fluid based on the difference in pressure of the power injection protocol between the vacuum fill state and the normal fill state.

In some non-limiting embodiments, when determining the first pressure of the power injection protocol when the power injection protocol is in the vacuum fill state, the at least one processor may be programmed or configured to determine a steady state force exerted on at least one force component of a pump (e.g., a piston or a rotor) when the power injection protocol is in the vacuum fill state and determine a speed of movement of the force component of the pump when the power injection protocol is in the vacuum fill state. In some non-limiting embodiments, when determining the second pressure of the power injection protocol when the power injection protocol is in the normal fill state, the at least one processor may be programmed or configured to determine a steady state force exerted on at least one force component of the pump when the power injection protocol is in the normal fill state and determine a speed of movement of the at least one force component of the pump when the power injection protocol is in the normal fill state. In some non-limiting embodiments, when determining the at least one characteristic of the power injection protocol, the at least one processor may be programmed or configured to determine a force exerted on at least one force component of a pump during each fill operation of a fluid reservoir of a plurality of fill operations of the fluid reservoir, wherein each fill operation of the fluid reservoir comprises an operation performed to fill the fluid reservoir with a medical fluid and determine a fill flow rate of the medical fluid through an orifice during each fill operation of the plurality of fill operations of the fluid reservoir. When determining the estimated value of viscosity of the medical fluid, the at least one processor may be programmed or configured to determine the estimated value of viscosity of the medical fluid based on the force exerted on the at least one force component of the pump during each fill operation of the plurality of fill operations of the fluid reservoir and the fill flow rate through the orifice during each fill operation of the plurality of fill operations of the fluid reservoir.

In some non-limiting embodiments, when determining the force exerted on the at least one force component of the pump during each fill operation of a plurality of fill operations of a fluid reservoir, the at least one processor may be programmed or configured to determine the force exerted on the force component of the pump during each fill operation of the plurality of fill operations of the fluid reservoir based on at least one of an amount of friction between the force component of the pump and a wall of the fluid reservoir, an area of the force component of the pump, a length of a tube component through which the medical fluid flows to the fluid reservoir, and a diameter of the tube component through which the medical fluid flows to the fluid reservoir. In some non-limiting embodiments, the at least one processor may be further programmed or configured to perform a linear regression calculation based on the force exerted on the at least one force component of the pump during each fill operation of the fluid reservoir of the plurality of fill operations of the fluid reservoir and the fill flow rate of the medical fluid through the orifice during each fill operation of the plurality of fill operations of the fluid reservoir, and when determining the estimated value of viscosity of the medical fluid, the at least one processor may be programmed or configured to determine the estimated value of viscosity of the medical fluid based on the linear regression calculation. In some non-limiting embodiments, when determining the at least one characteristic of the power injection protocol, the at least one processor may be programmed or configured to control at least one force component of a pump of the powered fluid injector to perform an operation to fill a fluid path and determine a fill time for the fluid path, wherein the fill time for the fluid path comprises an amount of time during which a volume of the fluid path is being filled with the medical fluid, and when determining the estimated value of viscosity of the medical fluid, the at least one processor may be programmed or configured to determine the estimated value of viscosity of the medical fluid based on the fill time for the fluid path.

In some non-limiting embodiments, when controlling the at least one force component of the pump of the powered fluid injector to fill the fluid path, the at least one processor may be programmed or configured to control the force component of the pump of the powered fluid injector to generate a constant pressure during the operation to fill the fluid path, and when calculating the hydraulic resistance score, the at least one processor may be programmed or configured to calculate the hydraulic resistance score based on a value of at least one of the constant pressure, an area of the force component of the pump, a distance travelled by the force component of the pump during the operation to fill the fluid path, and the fill time for the fluid path. In some non-limiting embodiments, when determining the at least one characteristic of the power injection protocol, the at least one processor may be programmed or configured to control at least one force component of a pump of the powered fluid injector to provide fluid flow of the medical fluid in a lumen of a tube component of the fluid injection system, control the force component of the pump of the powered fluid injector to alter at least one condition of fluid flow of the medical fluid in the lumen of the tube component, and detect a characteristic associated with altering the at least one condition of fluid flow of the medical fluid in the lumen of the tube component, and when determining the estimated value of viscosity of the medical fluid, the at least one processor may be programmed or configured to determine the estimated value of viscosity of the medical fluid based on the characteristic associated with altering the at least one condition of fluid flow of the fluid in the lumen of the tube component.

In some non-limiting embodiments, when controlling the force component of the pump of the powered fluid injector to provide flow of fluid in the lumen of the tube component, the at least one processor may be programmed or configured to control the force component of the pump of the powered fluid injector to provide laminar flow of fluid in the lumen of the tube component, and when controlling the force component of the pump of the powered fluid injector to alter the at least one condition of flow of the medical fluid in the lumen of the tube component, the at least one processor may be programmed or configured to control the force component of the pump of the powered fluid injector to transition from laminar flow of fluid in the lumen of the tube component to turbulent flow of fluid in the lumen. In some non-limiting embodiments, when controlling the force component of the pump of the powered fluid injector to alter the at least one condition of fluid flow of the medical fluid in the lumen of the tube component, the at least one processor may be programmed or configured to control the force component of the pump of the powered fluid injector to induce cavitation in the medical fluid. In some non-limiting embodiments, when controlling the force component of the pump to alter the at least one condition of fluid flow of the medical fluid in the lumen of the tube component, the at least one processor may be programmed or configured to control the force component of the pump of the powered fluid injector to shear one or more air bubbles that are present in the medical fluid. In some non-limiting embodiments, when controlling the force component of the pump of the powered fluid injector to alter the at least one condition of fluid flow of the medical fluid in the lumen of the tube component, the at least one processor may be programmed or configured to control the force component of the pump of the powered fluid injector to create turbulence in the medical fluid. In some non-limiting embodiments, when determining the at least one characteristic of the power injection protocol, the at least one processor may be programmed or configured to determine a time interval during which fluid flows from a high pressure side of an active fluid control component to a low pressure side of the active fluid control component, and when determining the estimated value of viscosity of the medical fluid, the at least one processor is programmed or configured to determine the estimated value of viscosity of the medical fluid based on the time interval.

In this way, non-limiting embodiments of the present disclosure provide for improving motor control accuracy based on the use of gain scheduling. Using gain scheduling, gains may be adjusted to best accommodate the hydraulic resistance produced by components of or connected to a fluid injection system (e.g., connected to a powered fluid injector). In this way, control of a motor of a powered fluid injector may be improved, thereby improving the bolus quality and volume accuracy of the delivered medical fluid, radiological image quality and the quality of the resulting diagnosis or treatment. Knowledge of the both hydraulic resistance and set-point (e.g., target) flow rate allow prediction of time to steady state for both pressure and flow rate along with prediction of the values of steady-state value for the pressure and flow rate. If the steady-state pressure is defined at or near the beginning of a fluid injection procedure, the speed of a force component (e.g., a piston or rotor) can be controlled more precisely to avoid maximum or minimum pressure events, deliver fluids with more precision, and/or react to other events that may occur during the fluid injection procedure. Further, the fluid injection system may be able to predict whether a programmed protocol is achievable based on hydraulic resistance associated with properties of the various components of the fluid injector setup. For example, user feedback systems may be implemented to alert the user if the programmed protocol is calculated to exceed a programmed pressure limit prior to initiating the injection. Such systems and features would spare the patient from unnecessary contrast and/or radiation exposure by alerting the user that a fluid injection protocol is not achievable, rather than simply starting the injection.

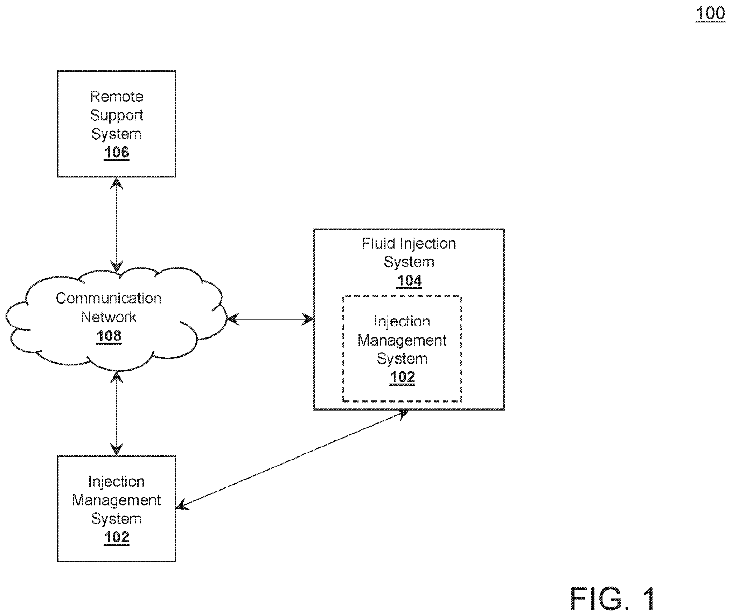

**FIG. 1** illustrates an embodiment of a diagram of an example environment 100 in which devices, systems, and/or methods, described herein, may be implemented. Environment 100 includes injection management system 102, fluid injection system 104, remote support system 108, and communication network 110. In some non-limiting embodiments, injection management system 102, fluid injection system 104, and/or remote support system 108, may interconnect (e.g., establish a connection to communicate) via wired connections, wireless connections, or a combination of wired and wireless connections.

In some non-limiting embodiments, injection management system 102 may include one or more devices capable of being in communication with one or more of fluid injection system 104, and/or remote support system 108, via communication network 110. For example, injection management system 102 may include a computing device, such as a computer, a server, a group of servers, and/or other like devices. In some non-limiting embodiments, injection management system 102 may be configured to receive data associated with an injection system (e.g., fluid injection system 104), generate a uniform resource locator (URL), and/or generate a QR code based on the URL. In some non-limiting embodiments, injection management system 102 may be a component of fluid injection system 104.

In some non-limiting embodiments, fluid injection system 104 may include one or more devices capable of being in communication with injection management system 102, and/or remote support system 108 via communication network 110. For example, fluid injection system 104 can include a computing device, such as one or more computers, a server, a group of servers, and/or other like devices. In some non-limiting embodiments, fluid injection system 104 includes one or more injection devices (e.g., one or more fluid injection devices). In some non-limiting embodiments, fluid injection system 104 is configured to administer a contrast agent to a patient, and administer an aqueous fluid, such as saline, to a patient before, during, and/or after administering the contrast agent. For example, fluid injection system 104 can inject one or more prescribed dosages of contrast fluid directly into a patient's blood stream via a syringe and catheter. In some non-limiting embodiments, fluid injection system 104 may be configured to continually administer the aqueous fluid to a patient through a peripherally inserted central catheter (PICC) and catheter, and one or more prescribed dosages of contrast fluid may be introduced into the PICC and administered via the catheter to the patient. In some non-limiting embodiments, fluid injection system 104 is configured to inject a dose of contrast fluid followed by administration of a particular volume of the aqueous fluid or may be configured to administer a "dual-flow" mixture of a selected ratio of a mixture of contrast and saline.

In some non-limiting embodiments, fluid injection system 104 may include one or more exemplary injection systems or injectors that are disclosed in: U.S. Patent No. 6,643,537; U.S. Patent No. 7,094,216; U.S. Patent No. 7,556,619; U.S. Patent No. 8,337,456; U.S. Patent No. 8,147,464; and U.S. 8,540,698. In some non-limiting embodiments, suitable fluid injection systems include but are not limited to, CT fluid injection systems, CV angiography fluid injection systems, MRI fluid injection systems, and PET fluid injection systems, such as the MEDRAD® Stellant CT Injection System, the MEDRAD® Stellant FLEX CT Injection System, the MEDRAD® MRXperion MR Injection System, the MEDRAD® Mark 7 Arterion Injection System, the MEDRAD® Intego PET Infusion System, or the MEDRAD® Centargo CT Injection System, available from Bayer HealthCare LLC, Indianola, PA.

In some non-limiting embodiments, fluid injection system 104 may include a workstation device that includes one or more devices capable of being in communication with one or more of injection management system 102, fluid injection system 104, and/or remote support system 108 via communication network 110. In some non-limiting embodiments, the workstation device may include a computing device, such as one or more computers, including a desktop computer, a laptop, a tablet, smart phone, and/or the like. In some non-limiting embodiments, the workstation device may provide a control interface for controlling operation of fluid injection system 104, including providing inputs to fluid injection system 104. Additionally or alternatively, the workstation device may display operational parameters of fluid injection system 104 during operation (e.g., during real-time operation) of fluid injection system 104. In some non-limiting embodiments, the workstation device may provide interconnectivity between fluid injection system 104 and other devices or systems, such as a scanner device (not shown). In some non-limiting embodiments, the workstation device may include the Certegra® Workstation provided by Bayer HealthCare LLC.

In some non-limiting embodiments, remote support system 108 may include one or more devices capable of being in communication with injection management system 102 and/or fluid injection system 104 via communication network 110. For example, remote system 108 may include a computing device, such as a computer, a server (e.g., a web server), a group of servers, and/or other like devices. In some non-limiting embodiments, remote support system 108 may include a back-end system associated with injection management system 102 and/or fluid injection system 104. In some non-limiting embodiments, remote support system 108 may include a cloud computing system that stores data in an associated database. In some non-limiting embodiments, remote support system 108 may be capable of interacting with fluid injection system 104 to provide functionality, such as remote equipment service for fluid injection system 104 (e.g., VirtualCARE® remote equipment support service for injection systems provided by Bayer HealthCare LLC). In some non-limiting embodiments, remote support system 108 may be operated by or on behalf of an original equipment manufacturer (OEM) of fluid injection system 104 (e.g., an OEM of one or more components or devices of fluid injection system 104), a provider of fluid injection system 104, an imaging site or a hospital in which fluid injection system 104 is operated, a service technician assigned to fluid injection system 104, and/or the like.

In some non-limiting embodiments, communication network 110 may include one or more wired and/or wireless networks. For example, communication network 110 may include a cellular network a local area network (LAN), a private network, an ad hoc network, an intranet, the Internet, a fiber optic-based network, a cloud computing network, a short range wireless communication network (e.g., a Bluetooth network, a near field communication (NFC) network, etc.) and/or the like, and/or a combination of these or other types of networks.

The number and arrangement of systems, devices, and networks shown in **FIG. 1** are provided as an example.

There may be additional systems and/or devices, fewer systems and/or devices, different systems and/or devices, and/or differently arranged systems and/or devices than those shown in **FIG. 1.** Furthermore, two or more systems or devices shown in **FIG. 1** may be implemented within a single system or a single device, or a single system or a single device may be implemented as multiple, distributed systems or devices. Additionally, or alternatively, a set of systems or a set of devices (e.g., one or more systems, one or more devices, etc.) of environment 100 may perform one or more functions described as being performed by another set of systems or another set of devices of environment 100.

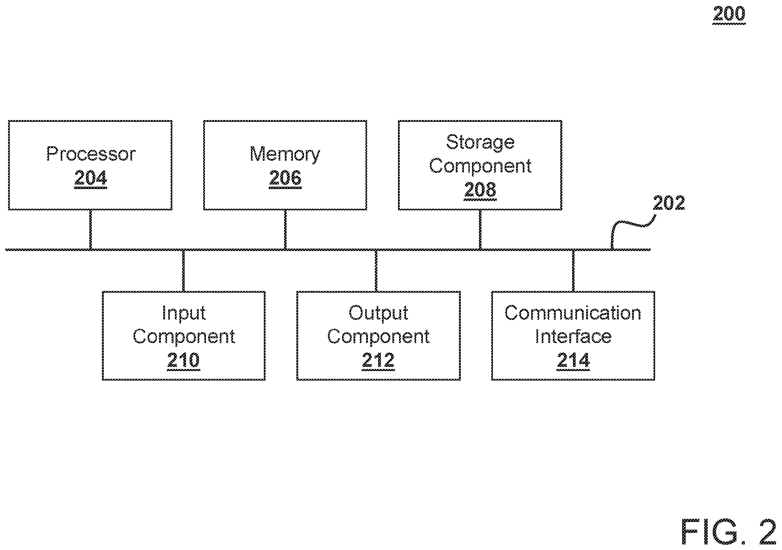

**FIG. 2** illustrates a diagram of example components of a device 200. Device 200 may correspond to one or more devices of injection management system 102, fluid injection system 104, and/or remote support system 108. In some non-limiting embodiments, injection management system 102, fluid injection system 104, and/or remote support system 108 can include at least one device 200 and/or at least one component of device 200. Device 200 may include one or more of bus 202, processor 204, memory 206, storage component 208, input component 210, output component 212, and communication interface 214.

Bus 202 may include a component that permits communication among the components of device 200. In some non-limiting embodiments, processor 204 may be implemented in hardware, software, or a combination of hardware and software. For example, processor 204 may include a processor, a microprocessor, a digital signal processor (DSP), and/or any processing component that can be programmed to perform a function. Memory 206 may include random access memory (RAM), read only memory (ROM), and/or another type of dynamic or static storage device (e.g., flash memory, magnetic memory, optical memory, etc.) that stores information and/or instructions for use by processor 204.

Storage component 208 may store information and/or software related to the operation and use of device 200. For example, storage component 208 may include a hard disk (e.g., a magnetic disk, an optical disk, a magneto-optic disk, a solid state disk, etc.), a compact disc (CD), a digital versatile disc (DVD), a floppy disk, a cartridge, a magnetic tape, and/or another type of computer-readable medium, along with a corresponding drive.

Input component 210 may include a component that permits device 200 to receive information, such as via user input (e.g., a touch screen display, a keyboard, a keypad, a mouse, a button, a switch, a microphone, etc.). Additionally, or alternatively, input component 210 may include one or more sensors for sensing information (e.g., an air sensor, a temperature sensor, a pressure sensor, an encoder, an accelerometer, a gyroscope, an actuator, etc.). Output component 212 may include a component that provides output information from device 200 (e.g., a display, a speaker, one or more light-emitting diodes (LEDs), etc.).

Communication interface 214 may include a transceiver-like component that enables device 200 to communicate with other devices, such as via a wired connection, a wireless connection, or a combination of wired and wireless connections. Communication interface 214 may permit device 200 to receive or deliver information from/to another device. For example, communication interface 214 may include an Ethernet interface, an optical interface, a coaxial interface, an infrared interface, a radio frequency (RF) interface, a universal serial bus (USB) interface, a Wi-Fi® interface, a cellular network interface, and/or the like.

Device 200 may perform one or more processes described herein. Device 200 may perform these processes based on processor 204 executing software instructions stored by a computer-readable medium, such as memory 206 and/or storage component 208.

Software instructions may be read into memory 206 and/or storage component 208 from another computer-readable medium or from another device via communication interface 214. When executed, software instructions stored in memory 206 and/or storage component 208 may cause processor 204 to perform one or more processes described herein. Additionally, or alternatively, hardwired circuitry may be used in place of or in combination with software instructions to perform one or more processes described herein. Thus, embodiments described herein are not limited to any specific combination of hardware circuitry and software.

The number and arrangement of components shown in **FIG. 2** are provided as an example. In some non-limiting embodiments, device 200 may include additional components, fewer components, different components, or differently arranged components than those shown in **FIG. 2.**

The present disclosure provides details with regard to estimating hydraulic resistance experienced by one or more components of a fluid injection system (e.g., fluid injection system 104) based on physical properties of the various components of the fluid injection system such as an internal geometry of components and length, such as a catheter, through which fluid flows during a fluid injection procedure and/or the viscosity of medical fluid used in the fluid injection procedure. In some non-limiting embodiments, pressure differentials generated by a powered fluid injector, and flow restrictions, including passive components and/or active components, may be used to measure fluid properties, such as fluid density, fluid viscosity, and/or air content (e.g., volume of air bubbles in a fluid).

In some non-limiting embodiments, physical properties of the fluid injection system may also be measured if the pressure and flow rate are known, such as catheter geometry (e.g., length, inner diameter, etc.), tubing geometry (e.g., length, diameter, volume, viscoelastic properties, etc.), and fluid reservoir characteristics (e.g., plunger friction against a wall of a syringe). The present disclosure utilizes syringe-based fluid injection systems for exemplary embodiments, but other methods of generating pressure, such as through peristaltic pumps, deformation of an elastic container, and/or the like also fall within the scope of the present disclosure.

In some non-limiting embodiments, a pressure-flow rate relationship in fluid injection system may be given by equation 1:

$$P_i \left[\frac{pound - force}{inch^2}\right] = R_{TOTAL,i} \left[\frac{pound - force - second}{inch^5}\right] \dot{Q}_{TOTAL,i} \left[\frac{inch^3}{second}\right] \quad \text{(Eq. 1)}$$

where the subscript $i$ indicates an instant in time, $P_i$ is pressure in a fluid reservoir (e.g., a syringe), $R_{TOTAL,i}$ is the total hydraulic resistance to fluid flow at time $i$, and $\dot{Q}_{TOTAL,i}$ is the volumetric flow rate at time $i$. The quantities in brackets are consistent units for each term. Further details may be found in International Patent Application No. PCT/US2018/048338.

In some non-limiting embodiments, the fluid injection system may generate distinct pressure differential waveforms over time. For example, the fluid injection system may generate a constant pressure waveform, a waveform with a linear ramp in pressure, a waveform with a step change in pressure, a polynomial change pressure waveform, an exponential change in pressure waveform, a sinusoidal variation pressure waveform, a pressure impulse waveform, and/or the like. As used herein, positive pressure may be pressure that is greater than atmospheric pressure and may include pressure that induces fluid flow from the fluid injector toward the patient. As used herein, negative pressure differential may include pressure that is less than atmospheric pressure and may include pressure that induces fluid flow toward fluid injection system 104.

According to various non-limiting embodiments, the fluid injection system may generate a pressure differential across a flow restriction, such as a reduction in inner diameter in a fluid flow path. Given a known hydraulic resistance of the flow restriction, a flow rate may be measured and used to determine one or more fluid properties (e.g., a characteristic of a power injection protocol) and/or physical properties of the fluid injection system. In some non-limiting embodiments, the flow restriction may be based on a passive component (e.g., a fill spike, disposable tubing, a J-tubes, a prime tube, a catheter, etc.) or may be based on an active component (e.g., a multi-position stopcock, a pinch valve, etc.). In some non-limiting embodiments, with active components, the hydraulic resistance may be controllable. For example, hydraulic resistance may be controllable by changing the inner diameter or area of an orifice of the active component through which fluid flows. In some non-limiting embodiments, a hydraulic resistance of a flow restriction may be unknown, and a known flow rate and pressure may be used to determine the hydraulic resistance. The resistance to flow of a fluid, $R_{TOTAL,i}$ is in part associated with viscous effects and in part related to inertial or density effects as shown by equation 2:

$$R_{TOTAL,i} = R_{viscous} + R_{inertial} \quad \text{(Eq. 2)}$$

In some non-limiting embodiments, hydraulic resistance may be due to the capillary effect of laminar, viscous flow in circular cross sections is represented by the Hagen-Poiseuille equation for fluid flow through a pipe as $R_{viscous}$ in equation 3:

$$R_{viscous} \left[\frac{pound - force - second}{inch^5}\right] = \frac{128\mu \left[\frac{pound - force - second}{inch^2}\right] L[inch]}{\pi D^4 [inch^4]} \quad \text{(Eq. 3)}$$

where $\mu \left[\frac{pound-force-second}{inch^2}\right]$ is the absolute viscosity, $L[inch]$ is the length of the flow restriction, and $D[inch]$ is the diameter of the flow restriction. Turbulent flow, which is a deviation from laminar flow, may cause equation 3 to become non-linear due to significant motion of fluid in directions at an angle or perpendicular to the tubing axis that consumes additional energy in moving the fluid through the tubing components and the catheter.

Density and inertial effects on hydraulic resistance may be associated with local flow restrictions or sudden changes in the fluid path cross-section, such as the entrance of the tubing components from the end of the fluid reservoir, transition from a first tubing component having a first inner diameter (ID) to a second tubing component having a second ID, or the distal opening of the catheter, which opens into the blood vessel of the patient. The component of hydraulic resistance is represented by $R_{inertial}$ in equation 4:

$$R_{inertial} \left[\frac{pound-force-second}{inch^5}\right] = \frac{8\rho \left[\frac{pound-force-second^2}{inch^2}\right]}{\pi^2 D^4 [inch^4] (Constant)^2} \dot{Q}_{TOTAL,i} \left[\frac{inch^3}{second}\right] \quad \text{(Eq. 4)}$$

where $\rho \left[\dfrac{pound-force-second^2}{inch^4}\right]$ is the density of the medical fluid in the fluid path and Constant is a term related to local geometry details. Since $R_{inertial}$ is a function of the volumetric flow rate, $\dot{Q}_{TOTAL,i}$, the overall relationship between pressure and flow rate (e.g., as defined by equation 1) is non-linear. Accordingly, change in a fluid flow cross-section may have an effect like that of turbulence based on molecular motion angled or perpendicular to the fluid path and resulting nonlinearity of equations 3 and 4. In some non-limiting embodiments, depending on geometry, $R_{inertial}$ and $R_{viscous}$ may have similar amplitude.

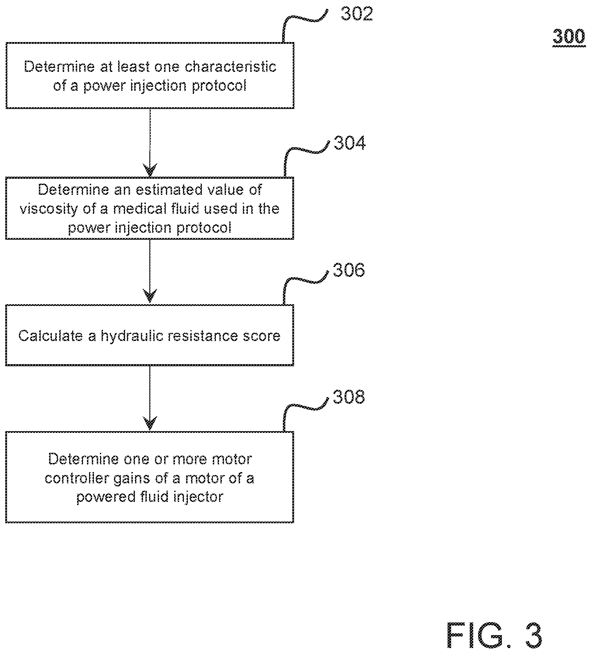

**FIG. 3** illustrates a flowchart of a non-limiting embodiment of a process 300 for controlling a fluid injection system. In some non-limiting embodiments, one or more of the steps of process 300 are at least partially performed by injection management system 102. In some non-limiting embodiments, one or more of the steps of process 300 may be at least partially performed by another device or a group of devices separate from or including injection management system 102, such as fluid injection system 104, and/or remote support system 108.

As shown in **FIG. 3,** at step 302, process 300 includes determining at least one characteristic of a power injection protocol, for example by injection management system 102. In some non-limiting embodiments, the at least one characteristic may include a steady state characteristic of a medical fluid flowing through a fluid path of fluid injection system 104. For example, the at least one characteristic may include a steady state characteristic of the medical fluid flowing through an orifice of the fluid path of fluid injection system 104. In some non-limiting embodiments, the medical fluid may include a flushing agent, contrast, and/or a combination thereof. In some non-limiting embodiments, injection management system 102 may determine at least one characteristic of the power injection protocol based on data received from one or more sensors, for example one or more sensors positioned on a component of fluid injection system 104, positioned within a component of fluid injection system 104, positioned on a fluid path of fluid injection system 104, positioned near an environment of fluid injection system 104, and/or the like. In some non-limiting embodiments, the data received from the one or more sensors may include operational parameters of fluid injection operations carried out by fluid injection system 104. In some non-limiting embodiments, operational parameters of fluid injection operations carried out by fluid injection system 104 may include one or more exemplary data types that are disclosed in U.S. Patent No. 7,457,804; U.S. Patent No. 7,996,381; U.S. Patent No. 8,521,716.

In some non-limiting embodiments, injection management system 102 may operate (e.g., advance, retract, rotate, etc.) a force component (e.g., a plunger, a piston, peristaltic pump, etc.) to maintain a constant pressure in a fluid path of fluid injection system 104. In some non-limiting embodiments, injection management system 102 may operate the force component at a constant rate to generate a constant flow rate in the fluid path of fluid injection system 104. In some non-limiting embodiments, when operating the force component at a constant pressure, injection management system 102 may measure a flow rate through a fluid path. In some non-limiting embodiments, when operating the force component to generate a constant flow rate in the fluid path, injection management system 102 may measure a pressure of the medical fluid in the fluid path.

In some non-limiting embodiments, injection management system 102 may determine at least one steady state characteristic of a contrast flowing through an orifice of the fluid path of fluid injection system 104. In some non-limiting embodiments, where an orifice is referred to there, the orifice may be substituted with a capillary tube and calculations may be performed in a same or similar manner. Additionally or alternatively, injection management system 102 may determine a steady state characteristic of saline flowing through the orifice of the fluid path of fluid injection system 104. In some non-limiting embodiments, injection management system 102 may calculate a characteristic ratio, which is a ratio of the steady state characteristic of saline to the steady state characteristic of the contrast.

In some non-limiting embodiments, injection management system 102 may determine a diameter of the orifice of the fluid path of fluid injection system 104. For example, injection management system 102 may determine the diameter of the orifice of the fluid path of fluid injection system 104 when a medical fluid flowing through the orifice reaches a steady state flow rate (e.g., a flow rate that remains the same when a diameter of the orifice is increased). In some non-limiting embodiments, injection management system 102 may store a value of the diameter of the orifice of the fluid path of fluid injection system 104 in a memory of injection management system 102. According to other embodiments, a diameter of the orifice may be entered into the fluid injection system, for example by a user or a scanning of a barcode or other information containing symbol or number associated with the fluid path.

In some non-limiting embodiments, the steady state characteristic of saline flowing through the orifice may include a steady state flow rate of saline flowing through the orifice based on a constant pressure of saline. Additionally, the steady state characteristic of the contrast flowing through the orifice may include a steady state flow rate of the contrast flowing through the orifice based on a constant pressure of the contrast. The characteristic ratio may include a flow rate ratio, which is a ratio of the steady state flow rate of saline to the steady state flow rate of the contrast. In some non-limiting embodiments, when calculating the characteristic ratio, injection management system 102 may calculate the flow rate ratio based on the steady state flow rate of saline and the steady state flow rate of the contrast.

In some non-limiting embodiments, the steady state characteristic of saline flowing through the orifice may include a steady state pressure of saline flowing through the orifice based on a constant flow rate of saline. Additionally, the steady state characteristic of the contrast flowing through the orifice may include a steady state pressure of the contrast flowing through the orifice based on a constant flow rate of the contrast. The characteristic ratio may include a pressure ratio, which is a ratio of the steady state pressure of saline to the steady state pressure of the contrast. In some non-limiting embodiments, when calculating the characteristic ratio, injection management system 102 may calculating the pressure ratio based on the steady state pressure of saline and the steady state pressure of the contrast.

In some non-limiting embodiments, injection management system 102 may determine a value of hydraulic capacitance of the medical fluid involved in a power injection protocol. For example, injection management system 102 may determine a value of hydraulic capacitance of the flushing agent (e.g., saline) and/or the contrast involved in a power injection protocol.

In some non-limiting embodiments, injection management system 102 may determine a first pressure of the power injection protocol when the power injection protocol is in a vacuum fill state, for example when the power injection protocol is closed off from receiving the fluid in a reservoir of fluid injection system 104. In some non-limiting embodiments, injection management system 102 may determine a second pressure of the power injection protocol when the power injection protocol is in a normal fill state, for example when the power injection protocol is open to receiving fluid in the reservoir of fluid injection system 104. In some non-limiting embodiments, when determining the first pressure of the power injection protocol when the power injection protocol is in the vacuum fill state, injection management system 102 may determine a steady state force exerted on a force component of a pump of a powered fluid injector when the power injection protocol is in the vacuum fill state and determine a speed of movement of the force component. In some non-limiting embodiments, when determining the second pressure of the power injection protocol when the power injection protocol is in the normal fill state, injection management system 102 may determine a steady state force exerted on a force component of the pump when the power injection protocol is in the normal fill state and determine a speed of movement of the force component. In some non-limiting embodiments, injection management system 102 may calculate a difference in pressure of the power injection protocol between the vacuum fill state and the normal fill state based on the first pressure and the second pressure. In some non-limiting embodiments, the pump of the powered fluid injector may include a piston configured for driving a plunger of a syringe and/or a peristaltic pump.

In some non-limiting embodiments, injection management system 102 may determine a force exerted on a force component (e.g., a piston, a plunger, a rotor, etc.) of a pump during each fill operation of a reservoir of fluid injection system 104 of a plurality of fill operations of the reservoir. Each fill operation of the reservoir comprises an operation performed to fill the reservoir with a fluid and injection management system 102 may determine a fill flow rate of the fluid through an orifice during each fill operation of the plurality of fill operations. In some non-limiting embodiments, when determining the force exerted on the force component of the pump during each fill operation of a plurality of fill operations of a reservoir, injection management system 102 may determine the force exerted on the force component of the pump during each fill operation of the plurality of fill operations based on an amount of friction between the force component of the pump and a wall of the reservoir (e.g., between a plunger and a wall of a syringe), an amount of friction between a medical fluid and a wall of the reservoir or a wall of a tube, an area of the force component of the pump, a length of a tube through which the fluid flows to the reservoir, and/or an inner diameter of the tube through which the fluid flows to the reservoir. In some non-limiting embodiments, injection management system 102 may perform a linear regression calculation based on the force exerted on the force component of the pump during each fill operation of the reservoir of the plurality of fill operations of the reservoir and the fill flow rate of the fluid through the orifice during each fill operation of the plurality of fill operations.

In some non-limiting embodiments, injection management system 102 may control a force component of a pump of the powered fluid injector of fluid injection system 104 to perform an operation to fill a fluid path and may determine a fill time for the fluid path. In some non-limiting embodiments, the fill time for the fluid path may include an amount of time during which a volume of the fluid path is being filled with a medical fluid (e.g., saline, contrast, etc.). In some non-limiting embodiments, when controlling the force component of the pump of the powered fluid injector to fill the fluid path, injection management system 102 may control the force component of the pump of the powered fluid injector to generate a constant pressure.

In some non-limiting embodiments, injection management system 102 may control a force component of a pump of the powered fluid injector of fluid injection system 104 to provide flow of the fluid in a lumen of a tube component (tubing, catheter, needle, etc.) of the fluid injection system, control the force component of the pump of the powered fluid injector of fluid injection system 104 to alter at least one condition of fluid flow of the fluid in the lumen of the tube component, and detect a characteristic associated with altering the at least one condition of fluid flow of the fluid in the lumen of the tube component. In some non-limiting embodiments, injection management system 102 may control the force component of the pump of the powered fluid injector to provide laminar flow of fluid in the lumen of the tube component. In some non-limiting embodiments, injection management system 102 may control the force component of the pump of the powered fluid injector to transition from laminar flow of fluid in the lumen to turbulent flow of fluid in the lumen of the tube component. In some non-limiting embodiments, injection management system 102 may control the force component of the pump of the powered fluid injector to induce cavitation in the medical fluid. In some non-limiting embodiments, injection management system 102 may control the force component of the pump of the powered fluid injector to shear one or more air bubbles that are present in the medical fluid. In some non-limiting embodiments, injection management system 102 may control the force component of the pump of the powered fluid injector to create turbulence in the medical fluid.

In some non-limiting embodiments, injection management system 102 may determine a time interval during which fluid flows from a high pressure side of an active fluid control component, such as a flow restriction or orifice, to a low pressure side of the active fluid control component.

As further shown in **FIG. 3,** at step 304, process 300 includes determining an estimated value of a viscosity of a medical fluid used in the power injection protocol. For example, injection management system 102 may determine the estimated value of viscosity of a medical fluid used in the power injection protocol based on the at least one characteristic of the power injection protocol. In some non-limiting embodiments, injection management system 102 may determine an estimated value of viscosity of the medical fluid based on a characteristic ratio (e.g., a ratio of a steady state characteristic of saline to a steady state characteristic of the contrast).

In some non-limiting embodiments, injection management system 102 may determine the estimated value of viscosity of the medical fluid based on a difference between a pressure when the power injection protocol is in a vacuum fill state and when the power injection protocol is in a normal fill state. In some non-limiting embodiments, injection management system 102 may determine the estimated value of viscosity of the medical fluid based on a force exerted on a force component of a pump of a power injector device during each fill operation of a plurality of fill operations of a fluid reservoir of fluid injection system 104 and a fill flow rate through an orifice of fluid injection system 104 during each fill operation of the plurality of fill operations of the fluid reservoir. In some non-limiting embodiments, injection management system 102 may determine the estimated value of viscosity of the medical fluid based on a linear regression calculation. In some non-limiting embodiments, injection management system 102 may determine the estimated value of viscosity of the medical fluid based on a fill time for a fluid path of fluid injection system 104. In some non-limiting embodiments, injection management system 102 may determine the estimated value of viscosity of the medical fluid based on a characteristic associated with altering at least one condition of fluid flow of the medical fluid in a lumen of a tube component of fluid injection system 104. In some non-limiting embodiments, injection management system 102 may determine the estimated value of viscosity of the medical fluid based on a time interval during which fluid flows from a high pressure side of an active fluid control component to a low pressure side of the active fluid control component.

In some non-limiting embodiments, injection management system 102 may determine the estimated value of viscosity of a medical fluid used in the power injection protocol based on a value of hydraulic capacitance of the medical fluid, for example, hydraulic capacitance of contrast and/or saline involved in a power injection protocol.

As further shown in **FIG. 3,** at step 306, process 300 includes calculating a hydraulic resistance score. For example, injection management system 102 may calculate the hydraulic resistance score based on the estimated value of viscosity of the medical fluid, such as the estimated value of viscosity of the medical fluid and a value of the diameter of the orifice of the fluid path of fluid injection system 104 (e.g., a value of the diameter of the orifice of the fluid path of fluid injection system 104 when a medical fluid flowing through the orifice reaches a steady state flow rate).

In some non-limiting embodiments, injection management system 102 may determine whether a characteristic ratio satisfies a threshold value and injection management system 102 may calculate the hydraulic resistance score based on determining that the characteristic ratio satisfies the threshold value. In some non-limiting embodiments, injection management system 102 may forego calculating the hydraulic resistance score based on determining that a characteristic ratio (e.g., a ratio of a steady state characteristic of saline to a steady state characteristic of the contrast) does not satisfy the threshold value. In some non-limiting embodiments, when determining whether the characteristic ratio satisfies the threshold value, injection management system 102 may determine whether the characteristic ratio satisfies (e.g., is greater than or equal to) a threshold value, e.g., a threshold value equal to 1.

In some non-limiting embodiments, injection management system 102 may calculate the hydraulic resistance score based on an estimated value of viscosity of contrast. In some non-limiting embodiments, injection management system 102 may calculate the hydraulic resistance score based on a value of at least one of the constant pressure (e.g., a constant pressure generated during an operation to fill a fluid path), an area of a force component of a pump of a powered fluid injector, a distance travelled by the force component of the pump during an operation to fill a fluid path, and/or a fill time for the fluid path.

As further shown in **FIG. 3,** at step 308, process 300 includes determining one or more motor controller gains of a motor of a powered fluid injector, for example, based on the hydraulic resistance score. Additionally or alternatively, injection management system 102 may determine one or more motor controller gains based on a hydraulic capacitance of a medical fluid involved in a power injection protocol.

In some non-limiting embodiments, injection management system 102 may implement the one or more motor controller gains on the motor of the pump of the powered fluid injector. For example, injection management system 102 may schedule the one or more motor controller gains and may instruct the motor of the pump to operate according to one or more of the one or more motor controller gains.

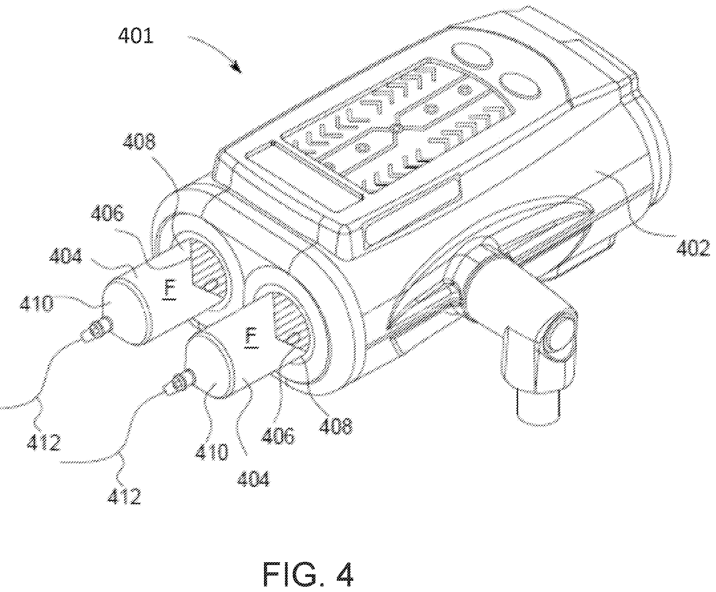

**FIG. 4** illustrates a diagram of a non-limiting embodiment of powered fluid injector 400, such as the MEDRAD® Stellant FLEX CT Injection System. In some non-limiting embodiments, powered fluid injector 400 may be the same or similar to fluid injection system 104. Injector head unit 401 may include housing 402 and at least one fluid reservoir 404, such as a syringe. In some non-limiting embodiments, powered fluid injector 400 may include, as shown in **FIG. 4,** a drive component (e.g., a force component) to control fluid flow into or out of fluid reservoir 404, such as a piston associated with each of syringes 404 that drives plunger 406 within a barrel of syringe 404. In some non-limiting embodiments, each of fluid reservoirs 404 is adapted to releasably interface with housing 402 at port 408. Each fluid reservoir 404 of powered fluid injector 400 is configured to be filled with at least one medical fluid, such as an imaging contrast media, saline and/or the like. Each fluid reservoir 404 may be filled with a different medical fluid F. In some non-limiting embodiments, powered fluid injector 400 may be a multi-syringe injector, as shown, where several fluid reservoirs 404 may be oriented side-by-side or in another spatial relationship and are separately actuated by respective pistons associated with powered fluid injector 400.

In some non-limiting embodiments, powered fluid injector 400 may be used during a medical procedure to inject the at least one medical fluid F into the vasculature of a patient by driving plungers 406 associated with fluid reservoir 404 with a drive component. The drive component may move plunger 406 to expel the fluid F from fluid reservoir 404 into and through fluid path set 412 during a priming, purging and/or fluid delivery step. In some non-limiting embodiments, fluid path set 412 may include at least one tube or tube set configured to be in fluid communication with each fluid reservoir 404 to place each fluid reservoir 404 in fluid communication with a flexible administration tube and associated catheter for delivering the fluid F from each fluid reservoir 404 to the vascular access site.

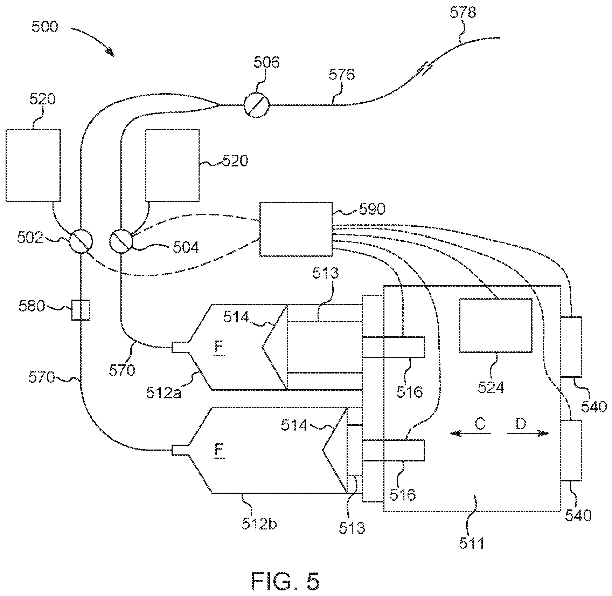

**FIG. 5** illustrates a schematic diagram of a non-limiting embodiment of a powered fluid injector 500. Powered fluid injector 500 may include housing 511 and one or more fluid reservoirs, such as syringes 512. Powered fluid injector 500 may include a drive component to control fluid flow into or out of a fluid reservoir, such as piston 513 associated with each of syringes 512 that drives plunger 514 within a barrel of syringe 512. Each piston 513 may be independently driven by an associated fluid actuator 516, such as a linear actuator, ball screw, lead screw, rack-and-pinion, pump roller, or the like.

The description of powered fluid injector 500 herein is generally directed to embodiments in which a fluid reservoir is a syringe 512 and the drive component for controlling fluid flow includes a piston 513 and plunger 514 operatively associated with the syringe 512. However, the present disclosure is to be understood as not limited to such embodiments. In some non-limiting embodiments, a fluid injection system may include a fluid pump as the fluid reservoir and a pump roller as the drive component. In some non-limiting embodiments, a fluid injection system considered and encompassed by the present disclosure may include a bag as the fluid reservoir and a compressive actuator configured to compress the bag as the drive component. As such, reference in this description to "syringe" is to be understood to encompass any type of fluid reservoir including syringes, fluid pumps, bags, and the like. Reference in this description to "piston", "plunger", and "piston actuator" are likewise understood to encompass any device operatively associated with fluid reservoir and configured for controlling fluid flow into and out of the fluid reservoir. In particular, the term "fluid actuator" may be used herein to encompass a device or devices operatively associated with fluid reservoir and configured for controlling fluid flow into and out of the fluid reservoir. Particular examples of a "fluid actuator" as used herein includes piston actuator 516 configured to actuate piston 513 by extending and retracting piston 513 within syringe 512, a pump rotor configured to actuate a fluid pump (e.g. a peristaltic pump) by compressing a tube associated with the fluid pump, and a compressive actuator configured to compress and/or squeeze a bag.

In some non-limiting embodiments, powered fluid injector 500 may be configured to deliver at least one medical fluid F, such as an imaging contrast media, saline, or any desired medical fluid, to a patient during an injection procedure. In some non-limiting embodiments, syringe 512 of powered fluid injector 500 may be configured to be filled with the at least one medical fluid F. Each syringe 512 may be filled with a different medical fluid F. The powered fluid injector 500 may be a multi-syringe injector, as shown, wherein several syringes 512 may be oriented side-by-side or in another spatial relationship and are separately actuated by respective pistons associated with powered fluid injector 500.

In some non-limiting embodiments, powered fluid injector 500 may be used during a medical procedure to inject at least one medical fluid F into the vasculature of a patient by driving plungers 514 associated with syringes 512 with pistons 513, respectively, generally as described for the powered fluid injector 400, described herein. Powered fluid injector 500 may include fluid path set 570 having at least one tube or tube set configured for fluid communication with each syringe 512 to place syringes 512 in fluid communication with administration line 576. A distal end of administration line 576 may be configured for fluid communication with catheter 578 configured for insertion into a vascular access site. As such, fluid communication may be established between syringes 512 and the patient such that medical fluid F can be injected from syringes 512 into the patient. Fluid path set 570 may have at least one sensor 580 positioned along a fluid path defined, for example, by fluid path set 570. The at least one sensor 580 may be placed on various other positions, such as the other fluid path of fluid path set 570, administration line 576, or at a region of the length of catheter 578. Sensor 580 may be configured to detect one or more operational parameters of fluid injection operations. In some non-limiting embodiments, sensor 580 may include an air sensor to detect whether air is present in the fluid path.

As further shown in **FIG. 5,** powered fluid injector 500 may include controller 590 for controlling actuation of pistons 513 via piston actuators 516, and for controlling other components of powered fluid injector 500. In some non-limiting embodiments, controller 590 may be the same or similar to injection management system 102. In some embodiments, controller 590 may be contained within housing 511. In some embodiments, controller 590 may be remotely mounted from housing 511, such as in a separate room from housing 511 so that an operator is not exposed to radiation during performance of a diagnostic procedure. In some embodiments, controller 590 may include multiple components (as described herein), of which some components are contained within housing 511 and some components are remotely mounted from housing 511.

In some non-limiting embodiments, powered fluid injector 500 may be configured to perform one or more injection procedures according to one or more injection protocols stored in a memory accessible by controller 590 or programmed into the controller 590. Prior to performing an injection procedure, air may be evacuated or purged from syringe 512 prior to connecting fluid path set 576 to catheter 578. During a purging operation, pistons 513 may be extended to a distalmost position in corresponding syringes 512 so that air is forced out of syringes 512. Syringes 512 and other portions of fluid path 570 may then be filled. During a filling operation, pistons 513 may be retracted proximally to draw medical fluid F from bulk fluid sources 520 into syringes 512, for example through valves 502, 504. During a priming operation, powered fluid injector 500 may be oriented with a head (e.g., housing 511) of powered fluid injector 500 facing upward with syringes 512 positioned vertically, allowing any air to accumulate at the distal ends of syringes 512. Pistons 513 may be extended distally to push against plungers 514 to remove air from syringes 512. Fluid path set 570 and administration line 576 may also be primed to remove air, for example, into a prime tube or bulk fluid sources 520. Once the purging, filling and priming operations are complete, administration line 576 may be connected to catheter 578 inserted into the patient, and the fluid injection protocol may be initiated.

As further shown in **FIG. 5,** powered fluid injector 500 may include user input devices 540 configured to allow manual control of pistons 513 and associated actuators 516. User input device 540 may include an electromechanical element, such as a rotatable knob, a rotatable dial, a lever, a slider, and/or the like. In some embodiments, each user input device 540 may include a touchscreen or microphone configured to receive voice commands. In some embodiments, each user input device 540 may be associated with one of syringes 512. Each user input device 540 may be in electrical communication with controller 590, such that, upon receiving at least one signal from user input device 540, controller 590 actuates associated piston 513 via an associated actuator 516.

In some non-limiting embodiments, user input devices 540 may be mounted or embedded at any location on housing 511 such as the back, side, top of the housing 11. In some embodiments, each user input devices 540 may be remotely mounted from housing 511, such as in a separate room from housing 511 so that the operator may control pistons 513 from a separate room not exposed to radiation during performance of a diagnostic procedure. In some embodiments, each user input device 540 may be mounted or embedded in a scanner (e.g. a CT, CV, PET, or MRI imaging device) configured for performing a diagnostic imaging procedure on the patient.

As further shown in **FIG. 5,** powered fluid injector 500 may include at least one user interface 524, such as a graphical user interface (GUI). User interface 124 may display information pertinent to a fluid injection procedure, such as injection status or progress, current flow rate, fluid pressure, and volume remaining in syringes 512 and in bulk fluid source 120 connected to powered fluid injector 500 and if a programmed injection protocol is determined to exceed parameters of powered fluid injector 500 according to the processes described herein. User interface 524 may be in electronic communication with controller 590 to allow a user to input parameters and control the processes of a fluid injection procedure.

Powered fluid injector 500 may further include valves 502, 504, 506 disposed at various locations along fluid path set 570. Each of valves 502, 504, 506 may be in the form of a shut-off valve, a stopcock, and/or a flow rate control valve to regulate flow of the medical fluid F to the patient. Valves 502 and 504 may be provided on fluid path set 570 between syringes 512 and bulk fluid sources 520. Valve 506 may be provided on fluid path set 570 downstream of valves 502 and 504. Each of valves 502, 504, 506 may be controllable by controller 590 or the user to regulate the flow of the fluid F through fluid path set 570. For example, any or all of valves 502, 504, 506 may be closed by controller 590 in response to detection of air in fluid path set 570. During a fill operation, valves 502 and 504 may be actuated by controller 590 to provide fluid communication between syringes 512 and bulk fluid sources 520, such that syringes 512 can draw medical fluid F from bulk fluid sources 520. Valves 502 and 504 may isolate syringes 512 and bulk fluid sources 520 from administration line 576 during a fill phase to prevent syringes 512 from drawing in fluid and/or air from the atmosphere. Valve 502 and 504 may also be selectively closed by controller 590 to prevent backflow of pressurized medical fluid F from fluid path set 570 into syringes 512 due to a difference in pressure and/or fluid viscosity between syringes 512 and fluid path set 570.

Further details and examples of suitable non-limiting powered injector systems, including syringes, controllers, air detectors, and/or fluid path sets are described in U.S. Patent Nos. 5,383,858; 7,553,294; 7,666,169; 8,945,051; 10,022,493; and 10,507,319.

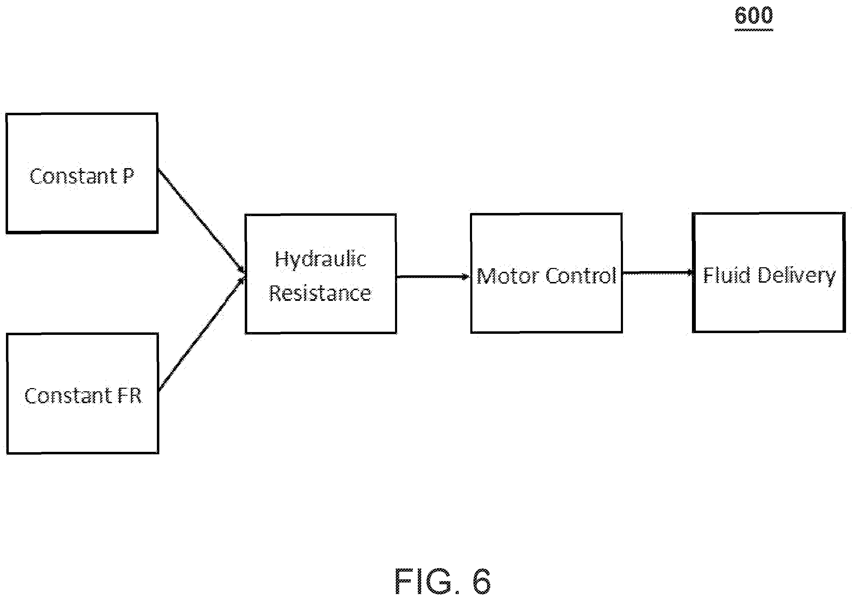

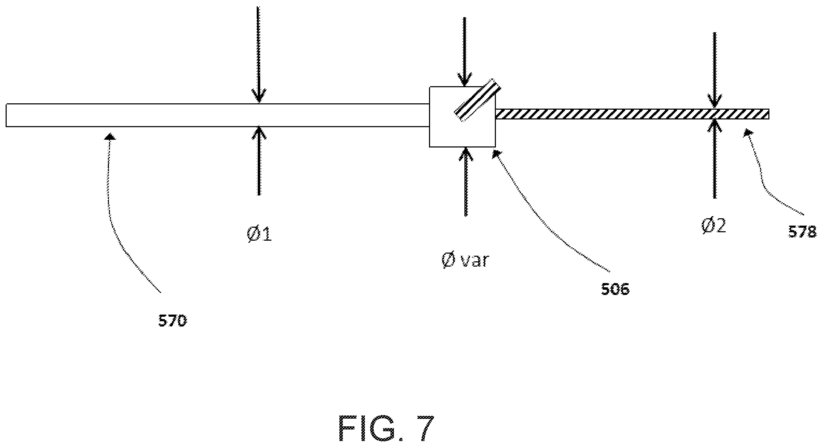

Further examples of non-limiting embodiments of the present disclosure are described with regard to powered fluid injector 500 shown in **FIG. 5.** In the description herein, syringe 512 may refer to either of syringe 512a or syringe 512b, unless otherwise specified. **FIG. 6** illustrates a diagram of a non-limiting embodiment of process flow 600 that provides details regarding a first example and a second example described below. Additionally, **FIG. 7** illustrates a diagram of a non-limiting embodiment of a portion of **FIG. 5,** which provides details regarding the first example and the second example described below.

In a first example, a variable diameter, $\varnothing_{var}$ as shown in **FIG. 7,** may be controlled by valve 506, which may be a stopcock, is used to measure a diameter, $\varnothing_2$, of catheter 578 by combining movements of valve 560 with movements of plunger 514 within a barrel of syringe 512. Although catheter length and shape may influence a value of the hydraulic resistance, catheter length and shape have a much smaller effect compared to the inner diameter of a catheter, as shown in equation 5, which is obtained by substituting equation 3 into equation 1:

$$\Delta P = R_{viscous} \left[ \frac{pound - force - second}{inch^5} \right] \dot{Q}_{TOTAL,i} \left[ \frac{inch^3}{second} \right]$$

$$= \frac{128\mu \left[ \frac{pound - force - second}{inch^2} \right] L[inch]}{\pi D^4 \left[ inch^4 \right]} \dot{Q}_{TOTAL,i} \left[ \frac{inch^3}{second} \right]$$

(Eqn. 5)

where $\Delta P$ is a pressure differential, $L$ is the length of tube 570 (e.g., a tube component, a catheter, etc.), $\mu$ is the dynamic fluid viscosity, $\dot{Q}_{TOTAL,i}$ is the volumetric flow rate, and D is the inner diameter ($\varnothing_1$, see **FIG. 7),** of tube 570. In the first example, it is assumed that valve 506 is actuated and first syringe 512a contains saline and second syringe 512b contains contrast. In the first example, valve 506 may start closed, disallowing any fluid flow along a fluid path defined by fluid path set 570, administration line 576, and catheter 578. Controller 590 may cause plunger 514 of first syringe 512a to move and generate a constant pressure (e.g., about 1-5 psi). In some non-limiting embodiments, the constant pressure may be maintained throughout a fluid injection procedure. Controller 590 may be able to detect the presence of air in the fluid path. The plunger displacement required to generate the constant pressure may be greater if air is contained in the fluid path as compared to a fluid path that has been fully primed with fluid, see e.g., International PCT Publication WO 2019/204605.

Controller 590 may then open valve 506 in small increments to allow fluid flow while injecting a first fluid, such as saline. While valve 506 is open, controller 590 may measure the flow rate, for example, by recording a change in a count of an encoder associated with the motor during a time interval, or other flow rate measurement device. When the flow rate remains the same for increasingly larger diameters of valve 506, this indicates that valve 506 has exceeded a diameter of catheter 578. Controller 590 may record the diameter of valve 506 at which a steady state flow rate of saline is detected.

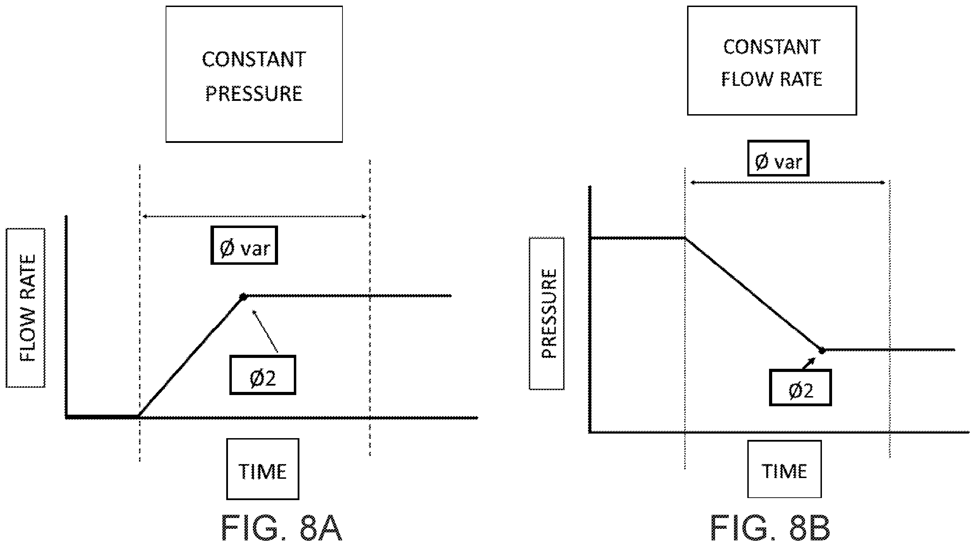

Referring to the first example, controller 590 may close valve 506 and relieve pressure with regard to the saline syringe 512a of powered fluid injector 500. Controller 590 may cause plunger 514 of second syringe 512b containing contrast to move and generate a constant pressure (e.g., about 1-5 psi). Controller 590 may then open valve 506 in small increments to allow flow of contrast. While valve 506 is open, controller 590 may measure the flow rate, by recording a change in a count of an encoder associated with the motor during a time interval. As shown by the graph of **FIG. 8A,** when the flow rate remains the same for increasingly larger diameters, $\varnothing_{var}$, of valve 506, this indicates that valve 506 has exceeded a diameter, $\varnothing_2$, of catheter 578. Controller 590 may record the diameter of valve 506 at which a steady state flow rate of contrast is detected.

Referring to the first example, controller 590 may close valve 506. Controller 590 may calculate a flow rate ratio of the steady state flow rate of saline to contrast, and the flow rate ratio may be used to provide an estimated value of viscosity of the contrast. In some non-limiting embodiments, controller 590 may determine the estimated value of viscosity of the contrast using a lookup table and/or an internal database using the flow rate ratio. In the case that flow rate ratio is equal to 1, controller 590 may determine that the same medical fluid is loaded into first syringe 512a and second syringe 512b. In the case that flow rate ratio is less than 1, controller 590 may determine that the medical fluid loaded into each of first syringe 512a and second syringe 512b is incorrect (e.g., contrast is contained in first syringe 512a and saline in second syringe 512b). In certain embodiments, the controller 590 may compare the estimated value of viscosity of contrast to the viscosity of known contrast agents and known concentrations; and determine the identity of the contrast being used in the injection protocol. In a dual flow-type protocol (i.e., where a programmed ratio of saline and contrast are mixed in the fluid line and injected), the controller 590 may compare the estimated value of viscosity of contrast to the known viscosities of saline/contrast solutions to determine if the correct ratio is being injected.

Referring to the first example, in certain embodiments, controller 590 may calculate a hydraulic resistance score based on the estimated value of viscosity of the contrast and the diameter of valve 506 at which a steady state flow rate of contrast was detected. Controller 590 may then determine one or more motor controller gains of a motor of a powered fluid injector 500. In some non-limiting embodiments, the one or more motor controller gains may be adjusted to pre-set values that are optimized based on a range of hydraulic resistance scores to achieve administration of accurate volumes of contrast and saline during the injection protocol.

In some non-limiting embodiments, controlling plunger 514 of syringe 512 under constant pressure may also allow for measurement of various viscoelastic properties of the fluid injection system, such as hydraulic capacitance (e.g., creep, stress relaxation, etc.). Hydraulic capacitance may refer to the tendency of elastic components, such as syringe walls, tube walls, etc., to deform under an applied constant stress. Further, hydraulic capacitance may be included as a component of hydraulic impedance where elastic components tend to expand when a fluid path is pressurized under pressures ranges from 100 psi up to 1200 psi. Because of the expansion elastic components, a volume of a fluid path that includes the elastic components increases, which may require a change (e.g., an increase in amplitude) to a control signal transmitted to the motor of powered fluid injector 500 to maintain a constant pressure and achieve accurate fluid delivery. Compliance of operation of powered fluid injector 500 for a power injection protocol and associated accuracy loss may therefore be recovered based on the change in the control signal over a small time period.

In a second example, the operation described with regard to the first example may be substantially the same, except that controller 590 may cause plungers 514 of syringes 512 to move and generate a constant flow rate. As shown by the graph of **FIG. 8B,** when the pressure remains the same for increasingly larger diameters, $\varnothing_{var}$, of valve 506, this indicates that valve 506 has exceeded a diameter, $\varnothing_2$, of catheter 578. Controller 590 may then measure pressures of the medical fluids as steady state delivery and a pressure ratio (e.g., a ratio of the steady state pressure of saline to the steady state pressure of the contrast) is used to provide an estimated value of viscosity of the contrast. Accuracy of the delivered volumes of fluids during the fluid injection protocol may then be corrected and optimized, as described herein. As shown in **FIG. 6,** with either constant pressure or constant flow rate, hydraulic resistance may be calculated, and motor control may be optimized based on the hydraulic resistance. By optimizing motor control, fluid delivery may therefore also be optimized.

In a third example, a negative pressure differential may be used during a fill operation of syringe 512 and fluid viscosity of the fill fluid determined. Referring to the third example, syringe 512 may be partially filled from bulk fluid supply 520 and air may be purged from fluid path set 570. The fluid path of powered fluid injector 500 may be closed off the system via valves 502, 504. Controller 590 may then retract plunger 514 via piston 513 such that a negative pressure differential is drawn on the fluid path of powered fluid injector 500. The force generated by piston 513 may be monitored controller 590 movement of piston 513 may be stopped when a steady state force has been reached. Controller 590 may record the speed of piston 513 and the steady state force. Controller 590 may relieve the negative pressure differential by returning piston 513 to the original starting position when the fluid path of powered fluid injector 500 was isolated. Controller 590 may open the fluid path to the bulk fluid supply 520 via valve 502 or valve 504 and retract plunger 514 to fill the remaining contents of syringe 512. Controller 590 may record the speed of piston 513 and the steady state filling force. Controller 590 may then calculate a difference in pressure between vacuum fill steady state and normal fill steady state to determine viscosity of the contrast. During the vacuum sequence, the fluid pressure may be known, which may be -14. 7 psi at sea level, therefore any load generated by piston 513 in addition to the vacuum force, which is the fluid pressure multiplied by the cross-sectional area of plunger 514 must be equal to an amount force to retract plunger 514 via piston 513 at that speed. The amount of force may be based on friction plus resistance of piston 513. As shown by equation 6:

$$F_R(v_p) = F_{vacuum} + F_{friction} + F_{resistance} = \left(14.7 * A_p\right) + F_{friction} + F_{resistance} \qquad \text{(Eqn. 6)}$$

where $F_R$ is the force to retract plunger 514 at a known speed $v_p$, $A_p$ is the cross-sectional area of plunger 514, $F_{friction}$ is the force associated with friction between plunger 514 and a wall of syringe 512, and $F_{resistance}$ is the resistance to filling due to geometry of tube components, fluid viscosity, and speed of piston 513. Equation 6 may be applied to both the vacuum and filling sequences, where $F_{resistance}$ is equal to 0 during the vacuum sequence since no fluid is flowing into syringe 512, permitting calculation of $F_{friction}$. In addition to the benefit to estimating hydraulic resistance, if the measured friction is significantly less than expected, this could indicate undesirable circumstances, for example, manufacturing problems, and/or that a user is attempting to re-use a syringe past recommended limits. During a subsequent filling sequence, equation 6 may be used to calculate $F_{resistance}$ using $F_{friction}$, which is known. Comparing the calculated value of $F_{resistance}$ to a calibration of this pressure drop with a viscosity of 1 cP allows for determining an estimated value of viscosity of contrast. Accuracy of the delivered volumes of fluids during the fluid injection protocol may then be corrected and optimized, as described herein. The pressure drop difference measured between saline and 20 cP contrast is approximately 5 psi.

In a fourth example, determining an estimated value of viscosity may be performed during a fill operation of syringe 512. In this way, the need to flow contrast to a patient while determining the estimated value of viscosity of contrast may be alleviated. The measured forces on plunger 514 during a fill sequence of syringe 512 are the friction force between plunger 514 and a wall of syringe 512 and the pressure differential on plunger 514 as shown in equation 7:

$$F_{Fill} = F_{Plunger\ friction} + F_{Fill\ \Delta P} \qquad \text{(Eqn. 7)}$$

Kinetic surface friction is, to the first order, not a function of velocity, and can be viewed as a constant at multiple flow rates during a filling operation of syringe 512. Assuming atmospheric pressure is the same in syringe 512 and on a backside of plunger 514, the pressure differential on plunger 514 may be modeled by the Hagen-Poiseuille equation, and the force exerted is equivalent to this pressure differential multiplied by the projected plunger area, $A_{Plunger}$, as shown in equation 8:

$$F_{Fill} = F_{Plunger\ friction} + \frac{128\mu L\dot{Q}}{\pi D^4} \cdot A_{Plunger} \qquad \text{(Eqn. 8)}$$

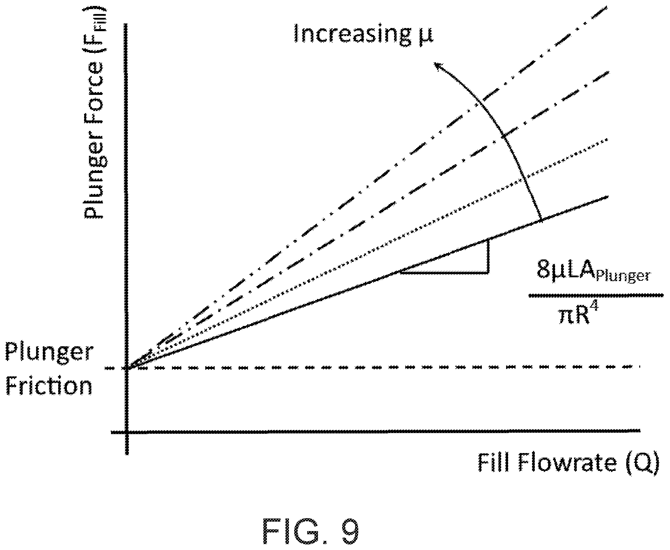

With a known length, L, of tube components involved in a fill sequence, fill tube internal diameter, *D,* and projected plunger area, $A_{Plunger}$, as well constant viscosity and plunger friction, equation 8 yields a linear relationship between fill plunger force and fill flow rate. The y-intercept of this line may be plunger frictional force and the slope of this line has all known constants, aside from viscosity. By filling syringe 512 at multiple flow rates and measuring the fill force, a plot of multiple lines representing plunger force versus fill flow rate can be generated. These calculations may all be performed by controller 590 and the results displayed or used in further algorithmic calculations. **FIG. 9** illustrates a diagram of a non-limiting embodiment of a plurality of plots of lines representing plunger force versus fill flow rate for values of viscosity, $\mu$, of contrast. A linear regression of the plunger force and flow rate plots shown in **FIG. 9** may provide a slope that can be used to determine an estimated value of viscosity of the contrast. The example reflects laminar flow but the example may be extended to non-linear and/or transient and/or non-laminar (e.g., turbulent) flow.

In a fifth example, one or more sensors to detect gross air (e.g., air sensors) may be used to determine an estimate of viscosity of the contrast. In one instance, two sensors may be used with powered fluid injector 500. A first air sensor may be positioned close to the distal end of syringe 512, and a second air sensor may be positioned distally down a fluid path (e.g., at a position at or near valve 506). The flow rate out of syringe *j* at time i, is represented by $\dot{Q}_{j,i}$ and may be related to displacement of piston 513 by equation 9:

$$\dot{Q}_{j,i} \left[ \frac{inch^3}{second} \right] = A_{0j,i} \frac{\Delta y_{j,i}}{\Delta t} \left[ \frac{inch^3}{second} \right] = \frac{P_i \left[ \frac{pound - force}{inch^2} \right]}{R_{TOTAL,i} \left[ \frac{pound - force - second}{inch^5} \right]} \qquad \text{(Eqn. 9)}$$

where $A_{0j,i}$ is the cross-sectional area of syringe 512 and plunger 514 at zero pressure and $\Delta y_{j,i}$ is the distance moved by plunger 514. Equation 9 assumes that minimal pressure is generated by the movement of piston, $\Delta y_{j,i}$. This condition is required because pressure deforms elastic components, such as tube components that are often used as part of the fluid path of powered fluid injector 500, such that elastic deformation may affect the diameter of the tube components. To determine an estimate of the viscosity of the contrast, plunger 514 may be driven to generate a constant pressure, as in the first example. The volume of the tube components is known, and the time to fill the volume between the air sensors may be measured. The measured time may be compared to a calibration curve between fill time and viscosity. The time to fill will increase with hydraulic resistance. One time constant, $\tau$, is the product of hydraulic resistance and hydraulic capacitance. Five time constants (e.g., $5\tau$) may be required to reach steady state, which the fill time plus the time to relieve pressure. The effects of hydraulic capacitance, such as the elastic deformation under stress, are also well characterized. Therefore, since pressure is measured, hydraulic resistance may be determined using equation 9.

Referring to the fifth example, empty syringes 512 may be installed in powered fluid injector 500 and then second syringe 512b may be filled with contrast. Controller 590 may close valve 506 and valves 502, 504 may be opened. Controller 590 may actuate plunger 514 of second syringe 512b to cause fluid to flow towards first syringe 512a. Controller 590 may determine an amount of time to fill the volume of the fluid path between second syringe 512b and first syringe 512a at a known flow rate. Controller 590 may determine an estimate of viscosity of the contrast based on the amount of time to fill the volume of the fluid path. A priming operation of powered fluid injector 500 may be carried out, for example to remove contrast from the saline fluid path and remove air.

In a sixth example, nonlinear pressure waveforms may be used to generate effects on fluid. The effects may include transitioning fluid flow from laminar flow to turbulent flow. In this way, the flow rate at the turbulent transition is related to fluid viscosity. Turbulent flow may be detected using a sensor, such as an accelerometer to measure vibration. The sudden change in the fluid velocity vector is acceleration and may be used to identify an inflection point between turbulent flow and laminar flow on a pressure-flow rate curve. Equation 4 identifies the inertial hydraulic resistance due to a change in cross section, which may accelerate a fluid such that an accelerometer can measure the acceleration. The effects may further include inducing fluid cavitation to create detectable air bubbles. The pressure or flow rate at which cavitation occurs depends on fluid viscosity. The effects may further include shearing existing bubbles and use flow rate and an air detection signal to determine an estimate of viscosity. The flow rate at which bubbles shear within the fluid depends on fluid viscosity. The effects may further include causing the onset and persistence of turbulence with increasing flow rate.

In a seventh example, active fluid control components may be used to determine an estimate of viscosity of contrast. Active fluid control components may be desirable because active fluid control components allow for diverse combinations of plunger movements and fluid control movements. For example, a stopcock may be opened or closed under various pressure waveforms, including linear, polynomial, exponential, and step relationships between position and time. The time over which fluid flows out of an active fluid control component to relieve pressure may then be measured and related to fluid viscosity.

**Claims**

1. A method for controlling a fluid injection system (104), comprising:

    determining, with at least one processor (204), at least one characteristic of a power injection protocol, wherein the at least one characteristic of the power injection protocol is associated with a medical fluid (F) involved in the power injection protocol;
    determining, with the at least one processor (204), an estimated value of viscosity of the medical fluid based on the at least one characteristic of the power injection protocol;
    calculating, with the at least one processor (204), a hydraulic resistance score based on the estimated value of viscosity of the medical fluid; and
    determining, with the at least one processor (204), one or more motor controller gains of a motor of a powered fluid injector (500) in the power injection protocol based on the hydraulic resistance score,

    **characterized in that**
    determining the at least one characteristic of the power injection protocol comprises:

    determining a steady state characteristic of saline flowing through an orifice of a fluid path of the powered fluid injector (500);
    determining a steady state characteristic of a contrast flowing through the orifice; and
    calculating a characteristic ratio, wherein the characteristic ratio is a ratio of the steady state characteristic of saline to the steady state characteristic of the contrast,
    wherein determining the estimated value of viscosity of the medical fluid (F) comprises:
    determining an estimated value of viscosity of the contrast based on the characteristic ratio; and
    wherein calculating the hydraulic resistance score comprises:
    calculating the hydraulic resistance score based on the estimated value of viscosity of the contrast.

2. The method of claim 1, further comprising implementing the one or more motor controller gains on the motor of the powered fluid injector.

3. The method of claim 1 or 2, wherein the motor is a motor of a pump of the powered fluid injector (500), and wherein the pump of the powered fluid injector comprises a piston (513) configured for driving a plunger (514) of a syringe (512) or a peristaltic pump.

4. The method of any of claims 1-3, further comprising determining whether the characteristic ratio satisfies a threshold value;
wherein calculating the hydraulic resistance score comprises calculating the hydraulic resistance score based on determining that the characteristic ratio satisfies the threshold value, preferably wherein determining whether the characteristic ratio satisfies the threshold value comprises determining whether the characteristic ratio satisfies a threshold value equal to 1.

5. The method of any of claims 1-4, wherein the steady state characteristic of saline flowing through the orifice comprises a steady state flow rate of saline flowing through the orifice based on a constant pressure of saline;

    wherein the steady state characteristic of the contrast flowing through the orifice comprises a steady state flow rate of the contrast flowing through the orifice based on a constant pressure of the contrast;
    wherein the characteristic ratio comprises a flow rate ratio, wherein the flow rate ratio is a ratio of the steady state flow rate of saline to the steady state flow rate of the contrast; and
    wherein calculating the characteristic ratio comprises calculating the flow rate ratio based on the steady state flow rate of saline and the steady state flow rate of the contrast.

6. The method of any of claims 1-5, wherein the steady state characteristic of saline flowing through the orifice comprises a steady state pressure of saline flowing through the orifice based on a constant flow rate of saline;

wherein the steady state characteristic of the contrast flowing through the orifice comprises a steady state pressure of the contrast flowing through the orifice based on a constant flow rate of the contrast;

wherein the characteristic ratio comprises a pressure ratio, wherein the pressure ratio is a ratio of the steady state pressure of saline to the steady state pressure of the contrast; and

wherein calculating the characteristic ratio comprises calculating the pressure ratio based on the steady state pressure of saline and the steady state pressure of the contrast.

7. The method of any of claims 1-6, further comprising determining a value of hydraulic capacitance of the medical fluid (F) involved in the power injection protocol,

wherein determining the motor controller gains comprises determining the motor controller gains based on the value of hydraulic capacitance of the medical fluid (F) involved in the power injection protocol.

8. The method of claim 1, wherein determining the at least one characteristic of the power injection protocol comprises:

determining a first pressure of the power injection protocol when the power injection protocol is in a vacuum fill state, wherein when the power injection protocol is in the vacuum fill state, the power injection protocol is closed off from receiving the medical fluid (F) in a fluid reservoir (404);

determining a second pressure of the power injection protocol when the power injection protocol is in a normal fill state, wherein when the power injection protocol is in the normal fill state, the power injection protocol is open to receiving medical fluid (F) in the fluid reservoir (404); and

calculating a difference in pressure of the power injection protocol between the vacuum fill state and the normal fill state based on the first pressure of the power injection protocol and the second pressure of the power injection protocol;

wherein determining the estimated value of viscosity of the medical fluid (F) comprises determining the estimated value of viscosity of the medical fluid (F) based on the difference in pressure of the power injection protocol between the vacuum fill state and the normal fill state, preferably, wherein determining the first pressure of the power injection protocol when the power injection protocol is in the vacuum fill state comprises:

determining a steady state force exerted on a force component of a pump when the power injection protocol is in the vacuum fill state; and

determining a speed of movement of the force component of the pump when the power injection protocol is in the vacuum fill state, and

wherein determining the second pressure of the power injection protocol when the power injection protocol is in the normal fill state comprises:

determining a steady state force exerted on a force component of the pump when the power injection protocol is in the normal fill state; and

determining a speed of movement of the force component of the pump when the power injection protocol is in the normal fill state.

9. The method of claim 1, wherein determining the at least one characteristic of the power injection protocol comprises:

determining a force exerted on a force component of a pump during each fill operation of a fluid reservoir (404) of a plurality of fill operations of the fluid reservoir (404), wherein each fill operation of the fluid reservoir (404) comprises an operation performed to fill the fluid reservoir (404) with a medical fluid (F); and

determining a fill flow rate of the medical fluid (F) through an orifice during each fill operation of the plurality of fill operations of the fluid reservoir (404);

wherein determining the estimated value of viscosity of the medical fluid (F) comprises determining the estimated value of viscosity of the medical fluid (F) based on the force exerted on the force component of the pump during each fill operation of the plurality of fill operations of the fluid reservoir (404) and the fill flow rate through the orifice during each fill operation of the plurality of fill operations of the fluid reservoir (404),

preferably, wherein determining the force exerted on the force component of the pump during each fill operation of a plurality of fill operations of a fluid reservoir (404) comprises:

determining the force exerted on the force component of the pump during each fill operation of the plurality of fill operations of the fluid reservoir (404) based on at least one of an amount of friction between the force component of the pump and a wall of the fluid reservoir (404), an area of the force component of the pump, a length of a tube component through which the medical fluid (F) flows to the fluid reservoir (404), and a diameter of the tube component through which the medical fluid (F) flows to the fluid reservoir (404).

10. The method of claim 9, further comprising performing a linear regression calculation based on the force exerted on the force component of the pump during each fill operation of the fluid reservoir (404) of the plurality of fill operations of the fluid reservoir (404) and the fill flow rate of the medical fluid (F) through the orifice during each fill operation of the plurality of fill operations of the fluid reservoir (404);
wherein determining the estimated value of viscosity of the medical fluid (F) comprises determining the estimated value of viscosity of the medical fluid (F) based on the linear regression calculation.

11. The method of claim 1, wherein determining the at least one characteristic of the power injection protocol comprises:

controlling a force component of a pump of the powered fluid injector (500) to perform an operation to fill a fluid path; and
determining a fill time for the fluid path, wherein the fill time for the fluid path comprises an amount of time during which a volume of the fluid path is being filled with the medical fluid (F);
wherein determining the estimated value of viscosity of the medical fluid (F) comprises determining the estimated value of viscosity of the medical fluid (F) based on the fill time for the fluid path.

12. The method of claim 11, wherein controlling the force component of the pump of the powered fluid injector (500) to fill the fluid path comprises controlling the force component of the pump of the powered fluid injector (500) to generate a constant pressure during the operation to fill the fluid path; and
wherein calculating the hydraulic resistance score comprises calculating the hydraulic resistance score based on a value of at least one of the constant pressure, an area of the force component of the pump, a distance travelled by the force component of the pump during the operation to fill the fluid path, and the fill time for the fluid path.

13. The method of claim 1, wherein determining the at least one characteristic of the power injection protocol comprises:

controlling a force component of a pump of the powered fluid injector (500) to provide fluid flow of the medical fluid (F) in a lumen of a tube component of the fluid injection system (104);
controlling the force component of the pump of the powered fluid injector (500) to alter at least one condition of fluid flow of the medical fluid (F) in the lumen of the tube component; and
detecting a characteristic associated with altering the at least one condition of fluid flow of the medical fluid (F) in the lumen of the tube component;
wherein determining the estimated value of viscosity of the medical fluid (F) comprises determining the estimated value of viscosity of the medical fluid (F) based on the characteristic associated with altering the at least one condition of fluid flow of the medical fluid (F) in the lumen of the tube component.

14. The method of claim 13, wherein controlling the force component of the pump of the powered fluid injector (500) to provide fluid flow of the medical fluid (F) in the lumen of the tube component comprises controlling the force component of the pump of the powered fluid injector (500) to provide laminar fluid flow of fluid in the lumen of the tube component;
wherein controlling the force component of the pump of the powered fluid injector (500) to alter the at least one condition of fluid flow of the medical fluid (F) in the lumen of the tube component comprises controlling the force component of the pump of the powered fluid injector (500) to transition from laminar fluid flow of fluid in the lumen of the tube component to turbulent fluid flow of fluid in the lumen of the tube component.

15. The method of claim 13 or 14, wherein controlling the force component of the pump of the powered fluid injector (500) to alter the at least one condition of fluid flow of the medical fluid (F) in the lumen of the tube component comprises one or more of controlling the force component of the pump of the powered fluid injector (500) to induce cavitation in the medical fluid (F), controlling the force component of the pump of the powered fluid injector (500) to shear one or more air bubbles that are present in the medical fluid (F), and controlling the force component of the pump of the powered fluid injector (500) to create turbulence in the medical fluid (F).

16. The method of claim 1, wherein determining the at least one characteristic of the power injection protocol comprises determining a time interval during which fluid flows from a high pressure side of an active fluid control component to a low pressure side of the active fluid control component; and
wherein determining the estimated value of viscosity of the medical fluid comprises determining the estimated value of viscosity of the medical fluid based on the time interval.

**Patentansprüche**

1. Verfahren zum Steuern eines Fluidinjektionssystems (104), wobei das Verfahren Folgendes umfasst:

   Bestimmen mit wenigstens einem Prozessor (204) wenigstens einer Eigenschaft eines Leistungsinjektionsprotokolls, wobei die wenigstens eine Eigenschaft des Leistungsinjektionsprotokolls einem medizinischen Fluid (F) zugeordnet ist, das in das Leistungsinjektionsprotokoll einbezogen ist;
   Bestimmen mit dem wenigstens einen Prozessor (204) eines geschätzten Wertes der Viskosität des medizinischen Fluids basierend auf der wenigstens einen Eigenschaft des Leistungsinjektionsprotokolls;
   Berechnen mit dem wenigstens einen Prozessor (204) einer Punktzahl des hydraulischen Widerstands basierend auf dem geschätzten Wert der Viskosität des medizinischen Fluids; und
   Bestimmen mit dem wenigstens einen Prozessor (204) einer oder mehrerer Motorsteuereinheit-Verstärkungen eines Motors eines angetriebenen Fluidinjektors (500) im Leistungsinjektionsprotokoll basierend auf der Punktzahl des hydraulischen Widerstands,
   **dadurch gekennzeichnet, dass** das Bestimmen der wenigstens einen Eigenschaft des Leistungsinjektionsprotokolls Folgendes umfasst:

   Bestimmen einer stationären Eigenschaft einer Kochsalzlösung, die durch eine Öffnung eines Fluidwegs des angetriebenen Fluidinjektors (500) strömt;
   Bestimmen einer stationären Eigenschaft eines Kontrastmittels, das durch die Öffnung strömt; und
   Berechnen eines Eigenschaftsverhältnisses, wobei das Eigenschaftsverhältnis ein Verhältnis der stationären Eigenschaft der Kochsalzlösung zur stationären Eigenschaft des Kontrastmittels ist,
   wobei das Bestimmen des geschätzten Werts der Viskosität des medizinischen Fluids (F) Folgendes umfasst:

   Bestimmen eines geschätzten Wertes der Viskosität des Kontrastmittels basierend auf dem Eigenschaftsverhältnis; und
   wobei das Berechnen der Punktzahl des hydraulischen Widerstands Folgendes umfasst:
   Berechnen der Punktzahl des hydraulischen Widerstands basierend auf dem geschätzten Wert der Viskosität des Kontrastmittels.

2. Verfahren nach Anspruch 1, das ferner das Implementieren der einen oder der mehreren Motorsteuereinheit-Verstärkungen in dem Motor des angetriebenen Fluidinjektors umfasst.

3. Verfahren nach Anspruch 1 oder 2, wobei der Motor ein Motor einer Pumpe des angetriebenen Fluidinjektors (500) ist und wobei die Pumpe des angetriebenen Fluidinjektors Folgendes umfasst
   eine Kolbenstange (513), die konfiguriert ist, einen Kolben (514) einer Spritze (512) oder einer Peristaltikpumpe anzutreiben.

4. Verfahren nach einem der Ansprüche 1-3, das ferner das Bestimmen umfasst, ob das Eigenschaftsverhältnis einem Schwellenwert entspricht;
   wobei das Berechnen der Punktzahl des hydraulischen Widerstands das Berechnen der Punktzahl des hydraulischen Widerstands basierend auf der Bestimmung umfasst, dass das Eigenschaftsverhältnis dem Schwellenwert entspricht, wobei vorzugsweise das Bestimmen, ob das Eigenschaftsverhältnis dem Schwellenwert entspricht, das Bestimmen umfasst, ob das Eigenschaftsverhältnis einem Schwellenwert gleich 1 entspricht.

5. Verfahren nach einem der Ansprüche 1-4, wobei die stationäre Eigenschaft der durch die Öffnung strömenden Kochsalzlösung eine stationäre Durchflussrate der durch die Öffnung strömenden Kochsalzlösung basierend auf einem konstanten Druck der Kochsalzlösung umfasst;

   wobei die stationäre Eigenschaft des durch die Öffnung strömenden Kontrastmittels eine stationäre Durchflussrate des durch die Öffnung strömenden Kontrastmittels basierend auf einem konstanten Druck des Kontrastmittels umfasst;
   wobei das Eigenschaftsverhältnis ein Durchflussratenverhältnis umfasst, wobei das Durchflussratenverhältnis ein Verhältnis der stationären Durchflussrate der Kochsalzlösung zur stationären Durchflussrate des Kontrastmittels ist; und
   wobei das Berechnen des Eigenschaftsverhältnisses das Berechnen des Durchflussratenverhältnisses basierend auf der stationären Durchflussrate der Kochsalzlösung und der stationären Durchflussrate des Kontrastmittels umfasst.

6. Verfahren nach einem der Ansprüche 1-5, wobei die stationäre Eigenschaft der durch die Öffnung strömenden Kochsalzlösung einen stationären Druck der durch die Öffnung strömenden Kochsalzlösung basierend auf einer konstanten Durchflussrate der Kochsalzlösung umfasst; wobei die stationäre Eigenschaft des durch die Öffnung strömenden Kontrastmittels einen stationären Druck des durch die Öffnung strömenden Kontrastmittels basierend auf einer konstanten Durchflussrate des Kontrastmittels umfasst;

wobei das Eigenschaftsverhältnis ein Druckverhältnis umfasst, wobei das Druckverhältnis ein Verhältnis des stationären Drucks der Kochsalzlösung zum stationären Druck des Kontrastmittels ist; und
wobei das Berechnen des Eigenschaftsverhältnisses das Berechnen des Druckverhältnisses basierend auf dem stationären Druck der Kochsalzlösung und dem stationären Druck des Kontrastmittels umfasst.

7. Verfahren nach einem der Ansprüche 1-6, das ferner das Bestimmen eines Wertes der hydraulischen Kapazität des medizinischen Fluids (F) umfasst, das in das Leistungsinjektionsprotokoll einbezogen ist,
wobei das Bestimmen der Motorsteuereinheit-Verstärkungen das Bestimmen der Motorsteuereinheit-Verstärkungen basierend auf dem Wert der hydraulischen Kapazität des medizinischen Fluids (F) umfasst, das in das Leistungsinjektionsprotokoll einbezogen ist.

8. Verfahren nach Anspruch 1, wobei das Bestimmen der wenigstens einen Eigenschaft des Leistungsinjektionsprotokolls Folgendes umfasst:

Bestimmen eines ersten Drucks des Leistungsinjektionsprotokolls, wenn sich das Leistungsinjektionsprotokoll in einem Vakuumfüllzustand befindet, wobei, wenn sich das Leistungsinjektionsprotokoll in dem Vakuumfüllzustand befindet, das Leistungsinjektionsprotokoll vom Empfangen des medizinischen Fluids (F) in einem Fluidreservoir (404) abgesperrt ist;
Bestimmen eines zweiten Drucks des Leistungsinjektionsprotokolls, wenn sich das Leistungsinjektionsprotokoll in einem normalen Füllzustand befindet, wobei, wenn sich das Leistungsinjektionsprotokoll in dem normalen Füllzustand befindet, das Leistungsinjektionsprotokoll für das Empfangen eines medizinischen Fluids (F) in dem Fluidreservoir (404) offen ist; und
Berechnen eines Druckunterschieds des Leistungsinjektionsprotokolls zwischen dem Vakuumfüllzustand und dem normalen Füllzustand basierend auf dem ersten Druck des Leistungsinjektionsprotokolls und dem zweiten Druck des Leistungsinjektionsprotokolls;
wobei das Bestimmen des geschätzten Wertes der Viskosität des medizinischen Fluids (F) vorzugsweise das Bestimmen des geschätzten Wertes der Viskosität des medizinischen Fluids (F) basierend auf dem Druckunterschied des Leistungsinjektionsprotokolls zwischen dem Vakuumfüllzustand und dem normalen Füllzustand umfasst,
wobei das Bestimmen des ersten Drucks des Leistungsinjektionsprotokolls, wenn sich das Leistungsinjektionsprotokoll im Vakuumfüllzustand befindet, Folgendes umfasst:

Bestimmen einer auf eine Kraftkomponente einer Pumpe ausgeübten stationären Kraft, wenn sich das Leistungsinjektionsprotokoll im Vakuumfüllzustand befindet; und
Bestimmen einer Bewegungsgeschwindigkeit der Kraftkomponente der Pumpe, wenn sich das Leistungsinjektionsprotokoll im Vakuumfüllzustand befindet, und
wobei das Bestimmen des zweiten Drucks des Leistungsinjektionsprotokolls, wenn sich das Leistungsinjektionsprotokoll im normalen Füllzustand befindet, Folgendes umfasst:

Bestimmen einer stationären Kraft, die auf eine Kraftkomponente der Pumpe ausgeübt wird, wenn sich das Leistungsinjektionsprotokoll im normalen Füllzustand befindet; und
Bestimmen einer Bewegungsgeschwindigkeit der Kraftkomponente der Pumpe, wenn sich das Leistungsinjektionsprotokoll im normalen Füllzustand befindet.

9. Verfahren nach Anspruch 1, wobei das Bestimmen der wenigstens einen Eigenschaft des Leistungsinjektionsprotokolls Folgendes umfasst:

Bestimmen einer Kraft, die während jedes Füllvorgangs eines Fluidreservoirs (404) von mehreren Füllvorgängen des Fluidreservoirs (404) auf eine Kraftkomponente einer Pumpe ausgeübt wird, wobei jeder Füllvorgang des Fluidreservoirs (404) einen Vorgang umfasst, der ausgeführt wird, um das Fluidreservoir (404) mit einem medizinischen Fluid (F) zu füllen; und

Bestimmen einer Fülldurchflussrate des medizinischen Fluids (F) durch eine Öffnung während jedes Füllvorgangs der mehreren Füllvorgänge des Fluidreservoirs (404);

wobei das Bestimmen des geschätzten Wertes der Viskosität des medizinischen Fluids (F) das Bestimmen des geschätzten Wertes der Viskosität des medizinischen Fluids (F) basierend auf der Kraft, die während jedes Füllvorgangs der mehreren Füllvorgänge des Fluidreservoirs (404) auf die Kraftkomponente der Pumpe ausgeübt wird, und der Fülldurchflussrate durch die Öffnung während jedes Füllvorgangs der mehreren Füllvorgänge des Fluidreservoirs (404) umfasst,

wobei vorzugsweise das Bestimmen der Kraft, die während jedes Füllvorgangs mehrerer Füllvorgänge eines Fluidreservoirs (404) auf die Kraftkomponente der Pumpe ausgeübt wird, Folgendes umfasst:

Bestimmen der Kraft, die während jedes Füllvorgangs der mehreren Füllvorgänge des Fluidreservoirs (404) auf die Kraftkomponente der Pumpe ausgeübt wird, basierend auf wenigstens einem eines Betrags der Reibung zwischen der Kraftkomponente der Pumpe und einer Wand des Fluidreservoirs (404), einer Fläche der Kraftkomponente der Pumpe, einer Länge einer Schlauchkomponente, durch die das medizinische Fluid (F) zum Fluidreservoir (404) strömt, und eines Durchmessers der Schlauchkomponente, durch die das medizinische Fluid (F) zum Fluidreservoir (404) strömt.

10. Verfahren nach Anspruch 9, das ferner das Ausführen einer linearen Regressionsberechnung basierend auf der Kraft, die während jedes Füllvorgangs des Fluidreservoirs (404) der mehreren Füllvorgänge des Fluidreservoirs (404) auf die Kraftkomponente der Pumpe ausgeübt wird, und der Fülldurchflussrate des medizinischen Fluids (F) durch die Öffnung während jedes Füllvorgangs der mehreren Füllvorgänge des Fluidreservoirs (404) umfasst;

wobei das Bestimmen des geschätzten Wertes der Viskosität des medizinischen Fluids (F) das Bestimmen des geschätzten Wertes der Viskosität des medizinischen Fluids (F) basierend auf der linearen Regressionsberechnung umfasst.

11. Verfahren nach Anspruch 1, wobei das Bestimmen der wenigstens einen Eigenschaft des Leistungsinjektionsprotokolls Folgendes umfasst:

Steuern einer Kraftkomponente einer Pumpe des angetriebenen Fluidinjektors (500), um einen Vorgang zum Füllen eines Fluidwegs auszuführen; und

Bestimmen einer Füllzeit für den Fluidweg, wobei die Füllzeit für den Fluidweg eine Zeitdauer umfasst, während der ein Volumen des Fluidwegs mit dem medizinischen Fluid (F) gefüllt wird;

wobei das Bestimmen des geschätzten Wertes der Viskosität des medizinischen Fluids (F) das Bestimmen des geschätzten Wertes der Viskosität des medizinischen Fluids (F) basierend auf der Füllzeit für den Fluidweg umfasst.

12. Verfahren nach Anspruch 11, wobei das Steuern der Kraftkomponente der Pumpe des angetriebenen Fluidinjektors (500) zum Füllen des Fluidwegs das Steuern der Kraftkomponente der Pumpe des angetriebenen Fluidinjektors (500) umfasst, um während des Vorgangs, um den Fluidweg zu füllen, einen konstanten Druck zu erzeugen; und

wobei das Berechnen der Punktzahl des hydraulischen Widerstands das Berechnen der Punktzahl des hydraulischen Widerstands basierend auf einem Wert von wenigstens einem des konstanten Drucks, einer Fläche der Kraftkomponente der Pumpe, einer von der Kraftkomponente der Pumpe während des Vorgangs zum Füllen des Fluidwegs zurückgelegten Strecke und der Füllzeit für den Fluidweg umfasst.

13. Verfahren nach Anspruch 1, wobei das Bestimmen der wenigstens einen Eigenschaft des Leistungsinjektionsprotokolls Folgendes umfasst:

Steuern einer Kraftkomponente einer Pumpe des angetriebenen Fluidinjektors (500), um eine Fluidströmung des medizinischen Fluids (F) in einem Lumen einer Schlauchkomponente des Fluidinjektionssystems (104) bereitzustellen;

Steuern der Kraftkomponente der Pumpe des angetriebenen Fluidinjektors (500), um wenigstens einen Zustand der Fluidströmung des medizinischen Fluids (F) im Lumen der Schlauchkomponente zu ändern; und

Detektieren einer Eigenschaft, die dem Ändern des wenigstens einen Zustands der Fluidströmung des medizinischen Fluids (F) im Lumen der Schlauchkomponente zugeordnet ist;

wobei das Bestimmen des geschätzten Wertes der Viskosität des medizinischen Fluids (F) das Bestimmen des geschätzten Wertes der Viskosität des medizinischen Fluids (F) basierend auf der Eigenschaft umfasst, die dem Ändern des wenigstens einen Zustands der Fluidströmung des medizinischen Fluids (F) im Lumen der Schlauchkomponente zugeordnet ist.

**14.** Verfahren nach Anspruch 13, wobei das Steuern der Kraftkomponente der Pumpe des angetriebenen Fluidinjektors (500), um eine Fluidströmung des medizinischen Fluids (F) im Lumen der Schlauchkomponente bereitzustellen, das Steuern der Kraftkomponente der Pumpe des angetriebenen Fluidinjektors (500) umfasst, um eine laminare Fluidströmung des Fluids im Lumen der Schlauchkomponente bereitzustellen;

wobei das Steuern der Kraftkomponente der Pumpe des angetriebenen Fluidinjektors (500), um den wenigstens einen Zustand der Fluidströmung des medizinischen Fluids (F) im Lumen der Schlauchkomponente zu ändern, das Steuern der Kraftkomponente der Pumpe des angetriebenen Fluidinjektors (500) umfasst, um von einer laminaren Fluidströmung des Fluids m Lumen der Schlauchkomponente zu einer turbulenten Fluidströmung des Fluids im Lumen der Schlauchkomponente überzugehen.

**15.** Verfahren nach Anspruch 13 oder 14, wobei das Steuern der Kraftkomponente der Pumpe des angetriebenen Fluidinjektors (500), um den wenigstens einen Zustand der Fluidströmung des medizinischen Fluids (F) im Lumen der Schlauchkomponente zu ändern, eines oder mehrere des Steuerns der Kraftkomponente der Pumpe des angetriebenen Fluidinjektors (500), um Kavitation in dem medizinischen Fluid (F) zu verursachen, des Steuerns der Kraftkomponente der Pumpe des angetriebenen Fluidinjektors (500), um eine oder mehrere Luftblasen, die in dem medizinischen Fluid (F) vorhanden sind, abzuscheren, und des Steuerns der Kraftkomponente der Pumpe des angetriebenen Fluidinjektors (500), um Turbulenz in dem medizinischen Fluid (F) zu erzeugen, umfasst.

**16.** Verfahren nach Anspruch 1, wobei das Bestimmen der wenigstens einen Eigenschaft des Leistungsinjektionsprotokolls das Bestimmen eines Zeitintervalls umfasst, während dessen Fluid von einer Hochdruckseite einer aktiven Fluidsteuerkomponente zu einer Niederdruckseite der aktiven Fluidsteuerkomponente strömt; und

wobei das Bestimmen des geschätzten Wertes der Viskosität des medizinischen Fluids das Bestimmen des geschätzten Wertes der Viskosität des medizinischen Fluids basierend auf dem Zeitintervall umfasst.

## Revendications

**1.** Procédé pour commander un système d'injection de fluide (104), comprenant :

la détermination, avec au moins un processeur (204), d'au moins une caractéristique d'un protocole d'injection sous pression, dans lequel l'au moins une caractéristique du protocole d'injection sous pression est associée à un fluide médical (F) impliqué dans le protocole d'injection sous pression ;
la détermination, avec l'au moins un processeur (204), d'une valeur estimée de viscosité du fluide médical sur la base de l'au moins une caractéristique du protocole d'injection sous pression ;
le calcul, avec l'au moins un processeur (204), d'un score de résistance hydraulique sur la base de la valeur estimée de viscosité du fluide médical ; et
la détermination, avec l'au moins un processeur (204), d'un ou de plusieurs gains de dispositif de commande de moteur d'un moteur d'un injecteur de fluide électrique (500) dans le protocole d'injection sous pression sur la base du score de résistance hydraulique,
**caractérisé en ce que** la détermination de l'au moins une caractéristique du protocole d'injection sous pression comprend :

la détermination d'un état stable caractéristique de solution saline s'écoulant à travers un orifice d'un trajet de fluide de l'injecteur de fluide électrique (500) ;
la détermination d'un état stable caractéristique d'un contraste s'écoulant à travers l'orifice ; et
le calcul d'un rapport de caractéristiques, le rapport de caractéristiques étant un rapport de la caractéristique d'état stable de solution saline à la caractéristique d'état stable du contraste,
dans lequel la détermination de la valeur estimée de viscosité du fluide médical (F) comprend :

la détermination d'une valeur estimée de viscosité du contraste sur la base du rapport de caractéristiques ; et
dans lequel le calcul du score de résistance hydraulique comprend :
le calcul du score de résistance hydraulique sur la base de la valeur de viscosité estimée du contraste.

**2.** Procédé selon la revendication 1, comprenant en outre la mise en œuvre des un ou plusieurs gains de dispositif de commande de moteur sur le moteur de l'injecteur de fluide électrique.

**3.** Procédé selon la revendication 1 ou la revendication 2, dans lequel le moteur est un moteur d'une pompe de l'injecteur de fluide électrique (500), et la pompe de l'injecteur de fluide électrique comprenant un tige du piston (513) configuré pour entraîner un piston (514) d'une seringue (512) ou d'une pompe péristaltique.

4. Procédé selon l'une quelconque des revendications 1 à 3, comprenant en outre la détermination de si le rapport de caractéristiques satisfait une valeur seuil ;
dans lequel le calcul du score de résistance hydraulique comprend le calcul du score de résistance hydraulique sur la base de la détermination que le rapport de caractéristiques satisfait la valeur seuil, la détermination de si le rapport de caractéristiques satisfait la valeur seuil comprenant préférablement la détermination de si le rapport de caractéristiques satisfait une valeur seuil égale à 1.

5. Procédé selon l'une quelconque des revendications 1 à 4, dans lequel l'état stable caractéristique de solution saline s'écoulant à travers l'orifice comprend un débit d'état stable de solution saline s'écoulant à travers l'orifice sur la base d'une pression constante de solution saline ;

dans lequel l'état stable caractéristique du contraste s'écoulant à travers l'orifice comprend un débit d'état stable du contraste s'écoulant à travers l'orifice sur la base d'une pression constante du contraste ;
dans lequel le rapport de caractéristiques comprend un rapport de débits, le rapport de débits étant un rapport du débit d'état stable de solution saline au débit d'état stable du contraste ; et
dans lequel le calcul du rapport de caractéristiques comprend le calcul du rapport de débits sur la base du débit d'état stable de solution saline et du débit d'état stable du contraste.

6. Procédé selon l'une quelconque des revendications 1 à 5, dans lequel l'état stable caractéristique de solution saline s'écoulant à travers l'orifice comprend une pression d'état stable de solution saline s'écoulant à travers l'orifice sur la base d'un débit constant de solution saline ;

dans lequel l'état stable caractéristique du contraste s'écoulant à travers l'orifice comprend une pression d'état stable du contraste s'écoulant à travers l'orifice sur la base d'un débit constant du contraste ;
dans lequel le rapport de caractéristiques comprend un rapport de pressions, le rapport de pressions étant un rapport de la pression d'état stable de solution saline à la pression d'état stable du contraste ; et
dans lequel le calcul du rapport de caractéristiques comprend le calcul du rapport de pressions sur la base de la pression d'état stable de solution saline et de la pression d'état stable du contraste.

7. Procédé selon l'une quelconque des revendications 1 à 6, comprenant en outre la détermination d'une valeur de capacité hydraulique du fluide médical (F) impliqué dans le protocole d'injection sous pression,
dans lequel la détermination des gains de dispositif de commande de moteur comprend la détermination des gains de dispositif de commande de moteur sur la base de la valeur de capacité hydraulique du fluide médical (F) impliqué dans le protocole d'injection sous pression.

8. Procédé selon la revendication 1, dans lequel la détermination de l'au moins une caractéristique du protocole d'injection sous pression comprend :

la détermination d'une première pression du protocole d'injection sous pression lorsque le protocole d'injection sous pression est dans un état de remplissage vide, dans lequel lorsque le protocole d'injection sous pression est dans l'état de remplissage vide, le protocole d'injection sous pression est fermé à la réception du fluide médical (F) dans un réservoir de fluide (404) ;
la détermination d'une deuxième pression du protocole d'injection sous pression lorsque le protocole d'injection sous pression est dans un état de remplissage normal, dans lequel lorsque le protocole d'injection sous pression est dans l'état de remplissage normal, le protocole d'injection sous pression est ouvert à la réception de fluide médical (F) dans le réservoir de fluide (404) ; et
le calcul d'une différence de pression du protocole d'injection sous pression entre l'état de remplissage vide et l'état de remplissage normal sur la base de la première pression du protocole d'injection sous pression et de la deuxième pression du protocole d'injection sous pression ;
dans lequel la détermination de la valeur estimée de viscosité du fluide médical (F) comprend la détermination de la valeur estimée de viscosité du fluide médical (F) sur la base de la différence de pression du protocole d'injection sous pression entre l'état de remplissage vide et l'état de remplissage normal, préférablement,
dans lequel la détermination de la première pression du protocole d'injection sous pression lorsque le protocole d'injection sous pression est dans l'état de remplissage vide comprend :

la détermination d'une force d'état stable exercée sur une composante de force d'une pompe lorsque le protocole d'injection sous pression est dans l'état de remplissage vide ; et

la détermination d'une vitesse de déplacement de la composante de force de la pompe lorsque le protocole d'injection sous pression est dans l'état de remplissage vide ; et

dans lequel la détermination de la deuxième pression du protocole d'injection sous pression lorsque le protocole d'injection sous pression est dans l'état de remplissage normal comprend :

la détermination d'une force d'état stable exercée sur une composante de force de la pompe lorsque le protocole d'injection sous pression est dans l'état de remplissage normal ; et

la détermination d'une vitesse de déplacement de la composante de force de la pompe lorsque le protocole d'injection sous pression est dans l'état de remplissage normal.

9. Procédé selon la revendication 1, dans lequel la détermination de l'au moins une caractéristique du protocole d'injection sous pression comprend :

la détermination d'une force exercée sur une composante de force d'une pompe pendant chaque opération de remplissage d'un réservoir de fluide (404) d'une pluralité d'opérations de remplissage du réservoir de fluide (404), dans lequel chaque opération de remplissage du réservoir de fluide (404) comprend une opération réalisée pour remplir le réservoir de fluide (404) avec un fluide médical (F) ; et

la détermination d'un débit de remplissage du fluide médical (F) à travers un orifice pendant chaque opération de remplissage de la pluralité d'opérations de remplissage du réservoir de fluide (404) ;

dans lequel la détermination de la valeur estimée de viscosité du fluide médical (F) comprend la détermination de la valeur estimée de viscosité du fluide médical (F) sur la base de la force exercée sur la composante de force de la pompe pendant chaque opération de remplissage de la pluralité d'opérations de remplissage du réservoir de fluide (404) et du débit de remplissage à travers l'orifice pendant chaque opération de remplissage de la pluralité d'opérations de remplissage du réservoir de fluide (404),

préférablement, dans lequel la détermination de la force exercée sur la composante de force de la pompe pendant chaque opération de remplissage d'une pluralité d'opérations de remplissage d'un réservoir de fluide (404) comprend :

la détermination de la force exercée sur la composante de force de la pompe pendant chaque opération de remplissage de la pluralité d'opérations de remplissage du réservoir de fluide (404) sur la base d'au moins un élément parmi une quantité de frottement entre la composante de force de la pompe et une paroi du réservoir de fluide (404), une zone de la composante de force de la pompe, une longueur d'une composante de tube à travers laquelle le fluide médical (F) s'écoule vers le réservoir de fluide (404), et un diamètre de la composante de tube à travers laquelle le fluide médical (F) s'écoule vers le réservoir de fluide (404).

10. Procédé selon la revendication 9, comprenant en outre la réalisation d'un calcul de régression linéaire sur la base de la force exercée sur la composante de force de la pompe pendant chaque opération de remplissage du réservoir de fluide (404) de la pluralité d'opérations de remplissage du réservoir de fluide (404) et du débit de remplissage du fluide médical (F) à travers l'orifice pendant chaque opération de remplissage de la pluralité d'opérations de remplissage du réservoir de fluide (404) ;

dans lequel la détermination de la valeur estimée de viscosité du fluide médical (F) comprend la détermination de la valeur estimée de viscosité du fluide médical (F) sur la base du calcul de régression linéaire.

11. Procédé selon la revendication 1, dans lequel la détermination de l'au moins une caractéristique du protocole d'injection sous pression comprend :

la commande d'une composante de force d'une pompe de l'injecteur de fluide électrique (500) pour réaliser une opération pour remplir un trajet de fluide ; et

la détermination d'un temps de remplissage pour le trajet de fluide, le temps de remplissage pour le trajet de fluide comprenant une quantité de temps pendant laquelle un volume du trajet de fluide est en cours de remplissage avec le fluide médical (F) ;

dans lequel la détermination de la valeur estimée de viscosité du fluide médical (F) comprend la détermination de la valeur estimée de viscosité du fluide médical (F) sur la base du temps de remplissage pour le trajet de fluide.

12. Procédé selon la revendication 11, dans lequel la commande de la composante de force de la pompe de l'injecteur de fluide électrique (500) pour remplir le trajet de fluide comprend la commande de la composante de force de la pompe de l'injecteur de fluide électrique (500) pour générer une pression constante pendant l'opération pour remplir le trajet de fluide ; et

dans lequel le calcul du score de résistance hydraulique comprend le calcul du score de résistance hydraulique sur la base d'une valeur d'au moins un élément parmi la pression constante, une zone de la composante de force de la pompe, une distance parcourue par la composante de force de la pompe pendant l'opération pour remplir le trajet de fluide, et le temps de remplissage pour le trajet de fluide.

13. Procédé selon la revendication 1, dans lequel la détermination de l'au moins une caractéristique du protocole d'injection sous pression comprend :

la commande d'une composante de force d'une pompe de l'injecteur de fluide électrique (500) pour fournir un écoulement de fluide du fluide médical (F) dans une lumière d'une composante de tube du système d'injection de fluide (104) ;

la commande de la composante de force de la pompe de l'injecteur de fluide électrique (500) pour modifier au moins une condition d'écoulement de fluide du fluide médical (F) dans la lumière de la composante de tube ; et

la détection d'une caractéristique associée à la modification de l'au moins une condition d'écoulement de fluide du fluide médical (F) dans la lumière de la composante de tube ;

dans lequel la détermination de la valeur estimée de viscosité du fluide médical (F) comprend la détermination de la valeur estimée de viscosité du fluide médical (F) sur la base de la caractéristique associée à la modification de l'au moins une condition d'écoulement de fluide du fluide médical (F) dans la lumière de la composante de tube.

14. Procédé selon la revendication 13, dans lequel la commande de la composante de force de la pompe de l'injecteur de fluide électrique (500) pour fournir un écoulement de fluide du fluide médical (F) dans la lumière de la composante de tube comprend la commande de la composante de force de la pompe de l'injecteur de fluide électrique (500) pour fournir un écoulement de fluide laminaire de fluide dans la lumière de la composante de tube ;

dans lequel la commande de la composante de force de la pompe de l'injecteur de fluide électrique (500) pour modifier l'au moins une condition d'écoulement de fluide du fluide médical (F) dans la lumière de la composante de tube comprend la commande de la composante de force de la pompe de l'injecteur de fluide électrique (500) pour passer d'un écoulement de fluide laminaire de fluide dans la lumière de la composante de tube à un écoulement de fluide turbulent de fluide dans la lumière de la composante de tube.

15. Procédé selon la revendication 13 ou la revendication 14, dans lequel la commande de la composante de force de la pompe de l'injecteur de fluide électrique (500) pour modifier l'au moins une condition d'écoulement de fluide du fluide médical (F) dans la lumière de la composante de tube comprend un ou plusieurs éléments parmi la commande de la composante de force de la pompe de l'injecteur de fluide électrique (500) pour induire une cavitation dans le fluide médical (F), la commande de la composante de force de la pompe de l'injecteur de fluide électrique (500) pour cisailler une ou plusieurs bulles d'air qui sont présentes dans le fluide médical (F), et la commande de la composante de force de la pompe de l'injecteur de fluide électrique (500) pour créer une turbulence dans le fluide médical (F).

16. Procédé selon la revendication 1, dans lequel la détermination de l'au moins une caractéristique du protocole d'injection sous pression comprend la détermination d'un intervalle de temps pendant lequel le fluide s'écoule d'un côté haute pression d'une composante de commande de fluide actif vers un côté basse pression de la composante de commande de fluide actif ; et

dans lequel la détermination de la valeur estimée de viscosité du fluide médical comprend la détermination de la valeur estimée de viscosité du fluide médical sur la base de l'intervalle de temps.

EP 4 158 642 B1

FIG. 1

FIG. 2

EP 4 158 642 B1

302

Determine at least one characteristic
of a power injection protocol

300

304

Determine an estimated value of
viscosity of a medical fluid used in the
power injection protocol

306

Calculate a hydraulic resistance score

308

Determine one or more motor
controller gains of a motor of a
powered fluid injector

FIG. 3

FIG. 4

FIG. 5

FIG. 6

EP 4 158 642 B1

**FIG. 7**

CONSTANT
FLOW RATE

Ø var

Ø2

PRESSURE

TIME

FIG. 8B

CONSTANT
PRESSURE

Ø var

Ø2

FLOW RATE

TIME

FIG. 8A

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62704893
- US 2020121860 A1
- US 6643537 B
- US 7094216 B
- US 7556619 B
- US 8337456 B
- US 8147464 B
- US 8540698 B
- US 2018048338 W
- US 7457804 B
- US 7996381 B
- US 8521716 B
- US 5383858 A
- US 7553294 B
- US 7666169 B
- US 8945051 B
- US 10022493 B
- US 10507319 B
- WO 2019204605 A